(19) **Europäisches Patentamt** / **European Patent Office** / **Office européen des brevets**

(11) **EP 3 396 573 A2**

(12) # EUROPEAN PATENT APPLICATION
published in accordance with Art. 153(4) EPC

(43) Date of publication:
**31.10.2018 Bulletin 2018/44**

(21) Application number: **16860219.1**

(22) Date of filing: **26.10.2016**

(51) Int Cl.:
*G06F 19/22* (2011.01)   *G06F 19/18* (2011.01)
*G06F 19/28* (2011.01)

(86) International application number:
**PCT/KR2016/012108**

(87) International publication number:
**WO 2017/074036 (04.05.2017 Gazette 2017/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **26.10.2015 KR 20150148717**

(71) Applicant: **CIPHEROME**
**Seoul 08826 (KR)**

(72) Inventor: **KIM, Ju Han**
**Seoul 05502 (KR)**

(74) Representative: **J A Kemp**
**14 South Square**
**Gray's Inn**
**London WC1R 5JJ (GB)**

(54) **METHOD AND SYSTEM FOR SELECTING CUSTOMIZED DRUG USING GENOMIC NUCLEOTIDE SEQUENCE VARIATION INFORMATION AND SURVIVAL INFORMATION OF CANCER PATIENT**

(57) The present invention relates to a method and system for selecting a customized drug using information of cancer genomic nucleotide sequence variations and patient survival and, more specifically, to a method and system for selecting a customized anticancer therapeutic drug using variant information of a synthetic cancer survival gene among cancer genomic nucleotide sequence variations. The method and system for customized anticancer therapy of the present invention using information of cancer genomic mutations and patient survival or the evaluation of invasive or metastatic ability of cancer cells or tissues correspond to a technique to effectively select an anticancer therapeutic drug having a good therapeutic effect and prognosis according to the individual through the variation analysis of synthetic cancer survival pairs of genes, which is derived from the information of cancer genomic nucleotide sequence variations and cancer survival and metastasis, and the method and system of the present invention have high reliability and can provide related information promptly and simply.

Fig. 1

DNAH2_XIRP2 (0.03634,0.01910, 0.02141)

Both <= 0.3:1/12 death (8.3%)
DNAH2 only <= 0.3:7/15 death (46.7%)
XIRP2 only <= 0.3:11/28 death (39.3)
None <= 0.3:55/118 death (46.6%)

Year
SKCM

**EP 3 396 573 A2**

**Description**

[Technical Field]

The present invention relates to a method and system for selecting a customized drug using genomic nucleotide sequence variant information and survival information of cancer patients, and more specifically, to a method and system for selecting a customized anticancer therapeutic drug using synthetic cancer survival gene variant information among genomic nucleotide sequence variant information of cancer patients.

[Background Art]

Since biotechnology has been developed, the whole genome sequence of humans is currently analyzed to reach the stage of predicting individual diseases and providing customized disease prevention and treatment methods.
Instability and accumulated deformation of the genome have been established as the etiology of cancer due to the rapid development of genomics, and rapid development of high-speed mass analysis and novel information processing technology of genome result in rapid actual clinical applications in advanced countries.
Meanwhile, the accurate prediction of prognoses is one of the important parts in the treatment of cancer patients with primary tumors. These prognoses are not only determined based on general clinical variant factors such as age and pathologic opinions but also are determined based on molecular variant factors such as genomic variation or amplification. Expression levels of ER, PR, and HER2 protein have been representatively identified as significant prognostic factors for breast cancer, and this has also been applied to actual treatment. Further, the study of predicting the prognosis using the molecular profile of ovarian cancer has been disclosed in recent, and this study reported that prognoses of corresponding patients are different from each other according to mutations present in BRCA1 and BRCA2 genes which are known to be prognostic factors of breast cancer. This study is one of the earliest studies which confirmed that molecular profile in addition to the clinical variables may predict the prognosis of cancer patients and which suggested that the molecular genomic indicators can be applied to various types of cancer in various ways.
Recently, analysis data of various cancer genomes and their analysis results have been announced through projects such as The Cancer Genome Atlas (TCGA) and International Cancer Genome Consortium (ICGC), and many related papers have been published. Profile analysis data on genomes, transcripts, epigenomes, and the like have been now published for most major cancer types. They include various contents such as finding genes that cause cancer, finding biomarkers to help molecular classification of cancer, finding prognostic factors, finding treatment response indicators, and heterogeneity of cancer tissue and cancer genetic variation.
Most studies published so far have focused on the characterization and role of individual genes, and studies related on the therapeutic targets or prognostic indicators of cancer are mostly limited to individual genes and a single cancer type. However, it is not easy to apply these identified causal genes directly to therapeutic targets or new drug development. The results of only biological indicator-based cancer research are not applicable to the personalized medicine which reflects individual differences due to the complexity and heterogeneity of cancer, and thus it shows various limitations in actual clinical application.
Therefore, in order to overcome the limitations of the current cancer research using single biological indicators, it is strongly required to develop a customized method of diagnosis and treatment of cancers based on data-based customized chemotherapy drug selection method which directly utilizes comprehensive analysis information of individual genome nucleotide sequence variants.

[Disclosure]

[Technical Problem]

The present invention was developed in view of the issues as described above and provides to a method and system for providing information for selecting the customized anticancer therapeutic drug in which a synthetic cancer survival pair of genes is derived using the genomic mutant information and survival information of cancer patients, the genomic nucleotide sequence variant information is analyzed to select at least one mutant gene belonging to at least one synthetic cancer survival pair of genes, and at least one candidate drug is selected to inhibit at least one corresponding gene pairing with the selected at least one variant gene to constitute the synthetic cancer survival pair of genes.

[Technical Solution]

An aspect of the present invention provides a method of providing information for customized anticancer therapeutic drug selection using a genomic nucleotide sequence variation of cancer patient, the method including: determining gene nucleotide sequence variant information of at least one gene belonging to a synthetic cancer survival pair of genes from the genomic nucleotide sequence information of the cancer patient; and selecting at least one candidate drug which inhibits at least one corresponding gene pairing with at least one variant gene belonging to the synthetic cancer survival pair of genes from the nucleotide sequence variant information.

Another aspect of the present invention provides a system for selecting a customized anticancer therapeutic drug using genomic nucleotide sequence variant information of a cancer patient, the system including: a database in which information related to an anticancer therapeutic drug to be applied to cancer patients and a gene inhibited by the drug is searched or extracted; a communication unit accessible to the database; a cancer genomic nucleotide sequence analyzer; a drug selection information provider; and a display, in which the cancer genomic nucleotide sequence analyzer includes: a variant gene selector selecting at least one variant gene belonging to a synthetic cancer survival pair of genes; and a corresponding gene selector selecting at least one corresponding gene pairing with the relevant at least one variant gene constituting the synthetic cancer survival pair of genes, and in which the drug selection information provider provides anticancer therapeutic drug selection information for inhibiting the relevant at least one corresponding gene.

Still another aspect of the present invention provides a computer-readable medium including an executable module for executing the processor executing an operation including: selecting a synthetic cancer survival pair of genes from genomic nucleotide sequence information of a cancer patient; and selecting at least one candidate drug that inhibits at least one corresponding gene pairing with at least one variant gene belonging to the synthetic cancer survival pair of genes.

Yet another aspect of the present invention provides a method of providing information for predicting prognosis of a cancer patient, the method including calculating the number of at least one gene belonging to the synthetic cancer survival pair of genes from nucleotide sequence information of a cancer patient genome.

Yet another aspect of the present invention provides a system for selecting a customized anticancer therapeutic drug using genomic nucleotide sequence variant information of a cancer patient, the system including: a database in which information related to an anticancer therapeutic drug to be applied to cancer patients and a gene inhibited by the drug is searched or extracted; a communication unit accessible to the database; a cancer genomic nucleotide sequence analyzer; a drug selection information provider; and a display, in which the cancer genomic nucleotide sequence analyzer includes: a variant gene pair selector selecting at least one variant gene belonging to a synthetic cancer survival pair of genes; and a corresponding gene selector selecting at least one corresponding gene pairing with the relevant at least one variant gene constituting the synthetic cancer survival pair of genes, and in which the drug selection information provider provides drug selection information for increasing the number of synthetic cancer survival pairs of genes of the cancer patient.

Yet another aspect of the present invention provides a computer-readable medium including an executable module for executing the processor executing an operation including: selecting a synthetic cancer survival pair of genes from genomic nucleotide sequence information of a cancer patient; and selecting a candidate drug that increases the number of synthetic cancer survival pairs of genes among at least one candidate drug that inhibits at least one corresponding gene pairing with at least one variant gene belonging to the synthetic cancer survival pair of genes.

[Advantageous Effects]

The method and system for selecting a customized drug using genomic mutant information and survival information of cancer patients according to the present invention are techniques which can select an anticancer therapeutic drug with excellent therapeutic effect and prognosis by an individual to provide highly reliable relevant information quickly and simply through the nucleotide sequence variant analysis of the synthetic cancer survival pair of genes derived from the genomic mutant information and survival information.

Using the method and system according to the present invention, at least one variant gene belonging to a gene pair inducing synthetic cancer survival is selected, and at least one corresponding gene pairing with the relevant variant gene to constitute the synthetic cancer survival pair of genes is selected, thereby selecting at least one anticancer therapeutic drug that inhibits the corresponding gene so that it is possible to select a customized anticancer agent by an individual from several comparative drugs. By predicting drug effects or the risk of side effects in advance, it is possible to determine the priority, optimum combination or use of anticancer agents applied to individuals. Further, the combination of at least one variant genes found in a plurality of patients having the relevant cancer type by specific cancer type is selected from the combinations of variant genes belonging to the synthetic cancer survival pair of genes, thereby selecting a combination of at least one anticancer therapeutic drug, which is predicted to have a good prognosis and therapeutic effect in a large number of patients of the relevant cancer type in general, which is independent of the genome sequence analysis results of individual patients. This is a technique that can be used for the development and clinical application of combination chemotherapy specified by cancer types, which is highly reliable to provide relevant information quickly and simply.

Further, the method and system according to the present invention can be used to predict cancer prognosis by analyzing the frequency and distribution of nucleotide sequence variants of a synthetic cancer survival pair of genes for each individual. The frequency and distribution of nucleotide sequence variant for each individual of a somatic mutation and a synthetic cancer survival pair of genes are analyzed and thus are used to predict the prognosis of cancer. In addition, the frequency and distribution analysis of individual nucleotide sequence variants of synthetic cancer survival pair of genes and somatic mutation can be efficiently used to predict therapeutic drug response.

[Description of Drawings]

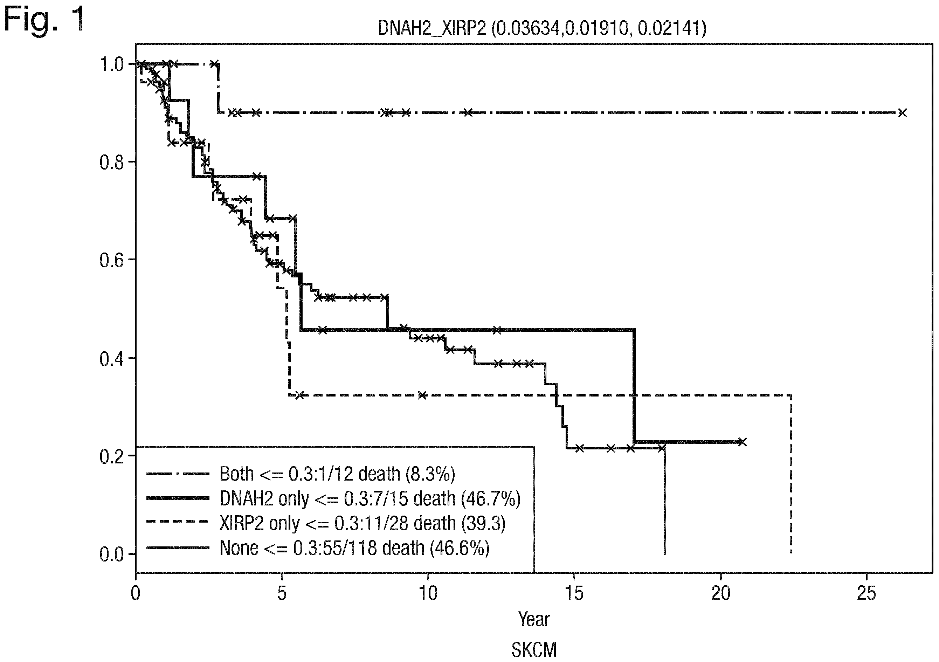

FIG. 1 illustrates a survival analysis curve in which a pair of DNAH2 and XIRP2 genes, which is one of the synthetic cancer survival pairs of genes found in a skin cutaneous melanoma patient is exemplified, both genes belonging to the corresponding synthetic cancer survival pair of genes have severe (low) gene deleteriousness scores (red line), one of the two genes has a severe gene deleteriousness score (yellow line and blue line), and neither gene does not have severe gene deleteriousness scores (green line).

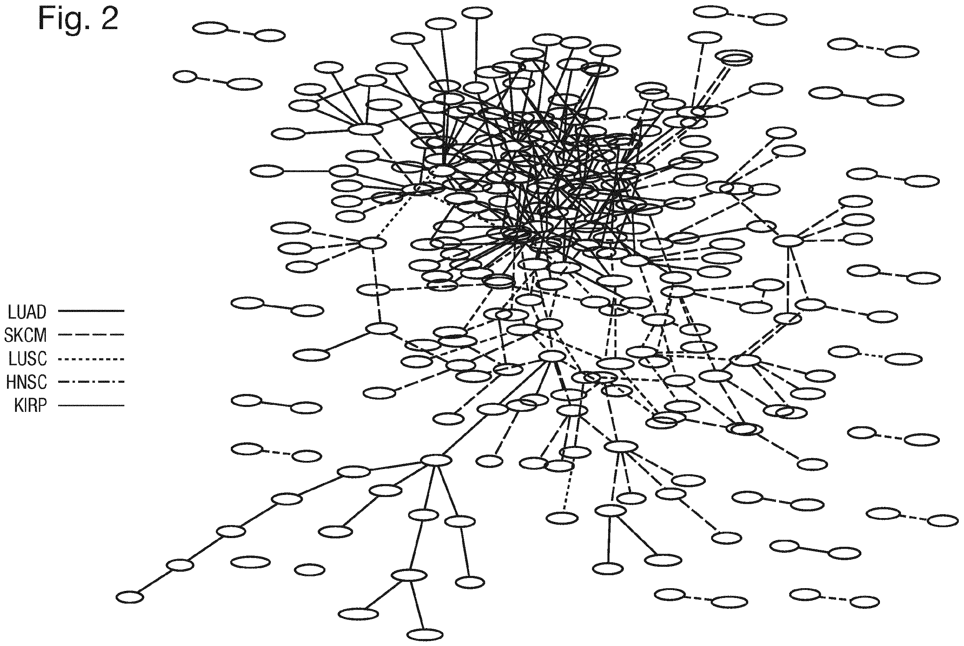

FIG. 2 illustrates a network of genes constituting a synthetic cancer survival pair of genes in which lung adenocarcinoma (LUAD) is represented by red line, skin cutaneous melanoma (SKCM) is represented by yellow line, lung squamous cell carcinoma (LUSC) is represented by blue line, head and neck squamous cell carcinoma (HNSC) is represented by brown line, and kidney renal clear cell carcinoma (KIRP) is represented by purple line.

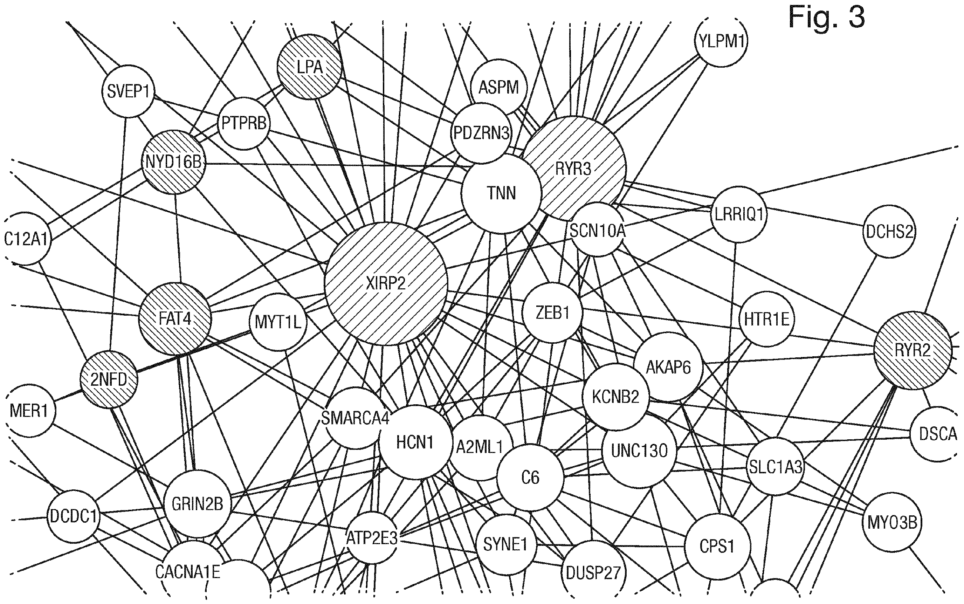

FIG. 3 is a drawing of overlaying a somatic mutation of a lung adenocarcinoma patient in the background of a lung adenocarcinoma synthetic cancer survival network composed of a synthetic cancer survival pair of genes found in a lung adenocarcinoma patient group. One node in the lung adenocarcinoma synthetic cancer survival network represented by gray color means one gene belonging to a synthetic cancer survival pair of genes of lung adenocarcinoma, a connection line connects between one synthetic cancer survival pair of genes, the yellow node and the red node represent genes showing a somatic mutation with a low gene deleteriousness score in the corresponding lung adenocarcinoma patient, the red node means a node constituting a synthetic cancer survival pair of genes together with the corresponding node connected by the connection line, the yellow node means a node that does not constitute a synthetic cancer survival pair of genes due to the absence of a gene having a low gene deleteriousness score among the corresponding nodes connected by the connection line.

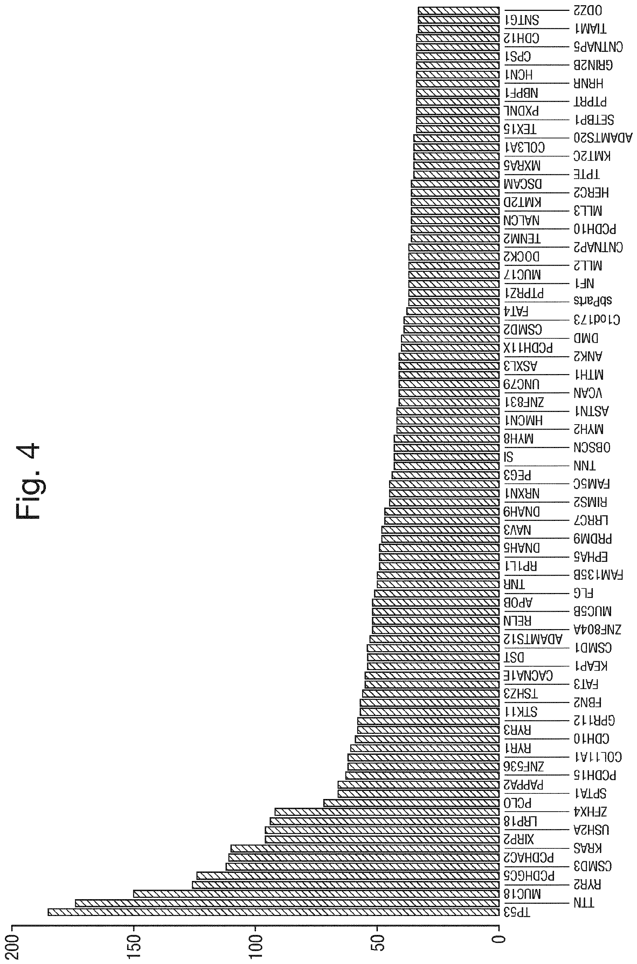

FIG. 4 is a bar graph in which lung adenocarcinoma is exemplified, and the occurrence frequency of a somatic mutation showing a low gene deleteriousness score in a lung adenocarcinoma patient is showed by each gene. It is shown that TP53 and TTN genes are most frequent gene deleteriousness somatic mutations.

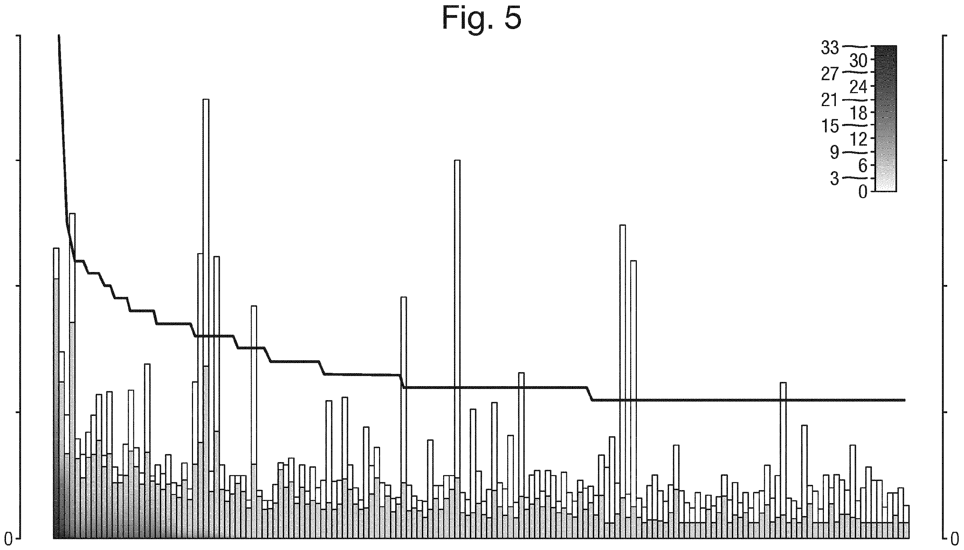

FIG. 5 is a cumulative bar graph in which lung adenocarcinoma is exemplified, the participation frequency how many times each of genes constituting a synthetic cancer survival pair of genes in a lung adenocarcinoma patient participates in synthetic cancer survival pairs of genes is shown. The exemplified red graph of broken lines is a view of exemplifying the frequency how many times the relevant gene participates in synthetic cancer survival pairs of genes. XIRP2 and RYR3 most frequently constitute synthetic cancer survival pairs of genes in lung adenocarcinoma.

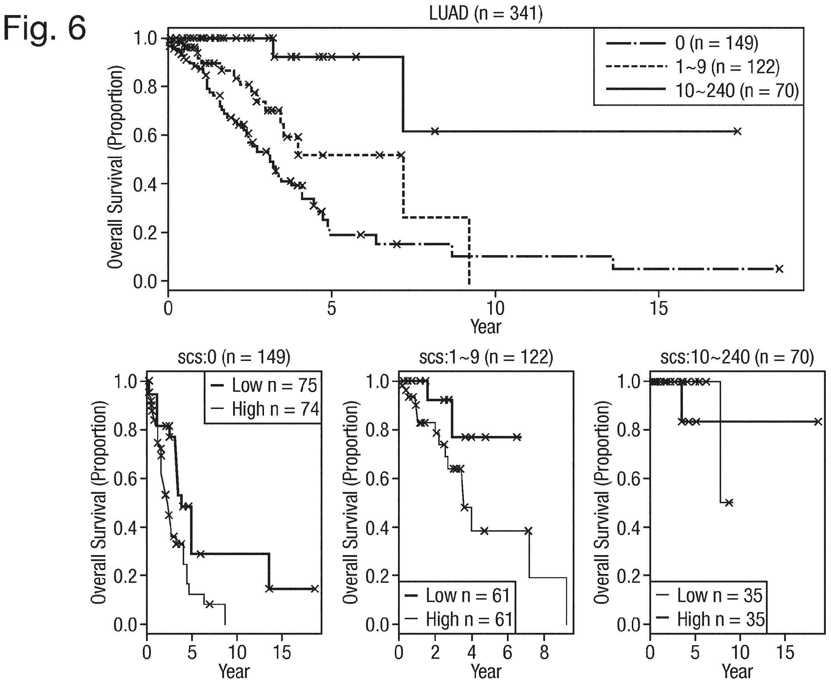

FIG. 6 illustrates the results of survival analysis by applying Cox proportional hazards model to a total of 341 patients with lung adenocarcinoma in which total patients are divided into 149 patients without any synthetic cancer survival pair of genes, 122 patients with more than 1 to less than 10 pairs, and 70 patients having more than 10 pairs. In the three survival analysis graphs at the bottom of FIG. 6, 341 lung adenocarcinoma patients are divided into total three groups according to the number of retained synthetic cancer survival pairs of genes, and each subgroup is divided into two groups according to high and low of the number of somatic mutations. Survival curves of 74 patients, 61 patients, and 35 patients with higher somatic mutation burdens are shown in red, and survival curves of 75 patients, 61 patients, and 35 patients with lower somatic mutation burdens are shown in sky blue.

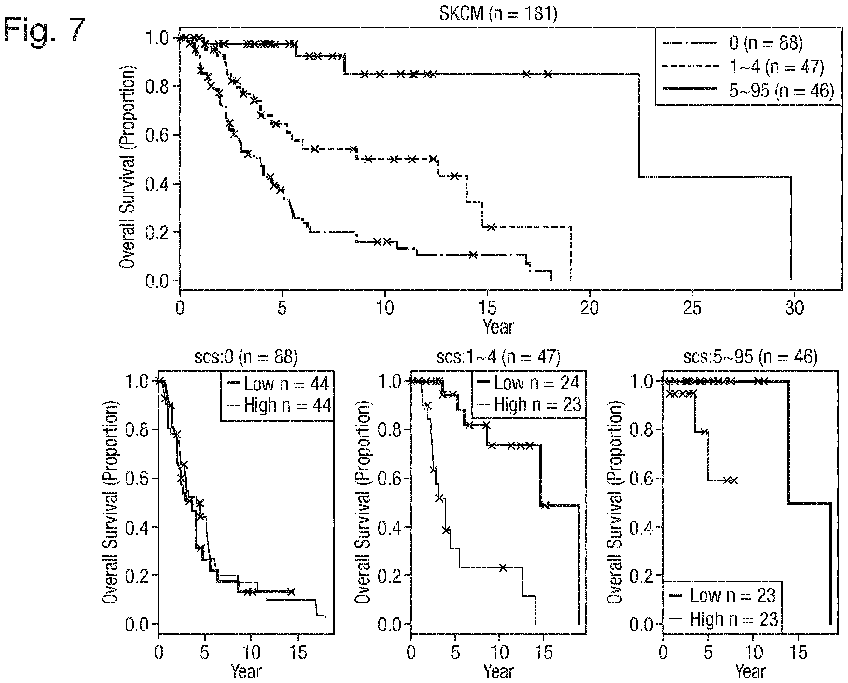

FIG. 7 illustrates the results of survival analysis by applying Cox proportional hazards model to a total of 181 patients with skin cutaneous melanoma in which total patients are divided into 88 patients without any synthetic cancer survival pair of genes, 47 patients with more than 1 to less than 5 pairs, and 46 patients having more than 5 pairs. In the three survival analysis graphs at the bottom of FIG. 7, 181 skin cutaneous melanoma patients are divided into total three groups according to the number of retained synthetic cancer survival pairs of genes, and each subgroup is divided into two groups according to high and low of the number of somatic mutations. Survival curves of 44 patients, 23 patients, and 23 patients with higher somatic mutation burdens are shown in red, and survival curves of 44 patients, 24 patients, and 23 patients with lower somatic mutation burdens are shown in sky blue.

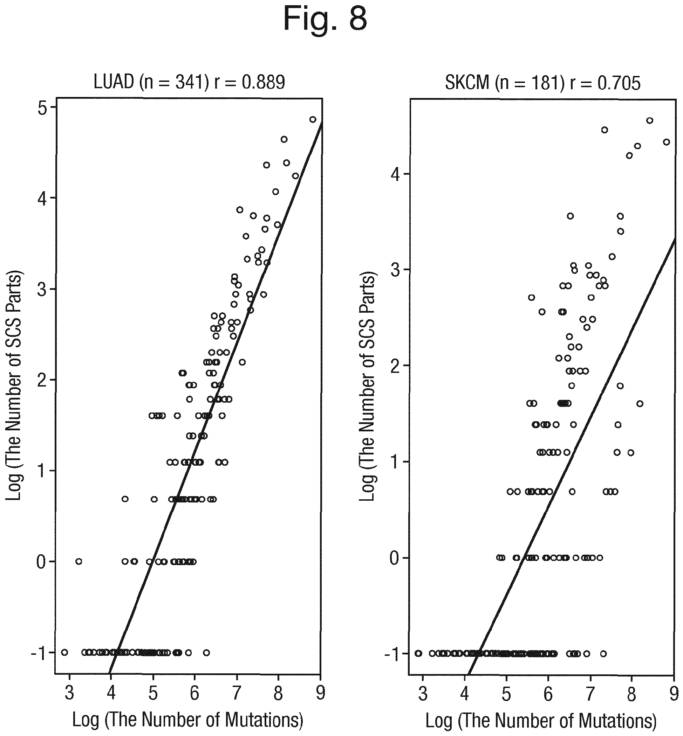

FIG. 8 is a graph illustrating a log-log relationship of the correlation between the somatic mutation burden and the synthetic cancer survival burden in lung adenocarcinoma patients and skin cutaneous melanoma patients.

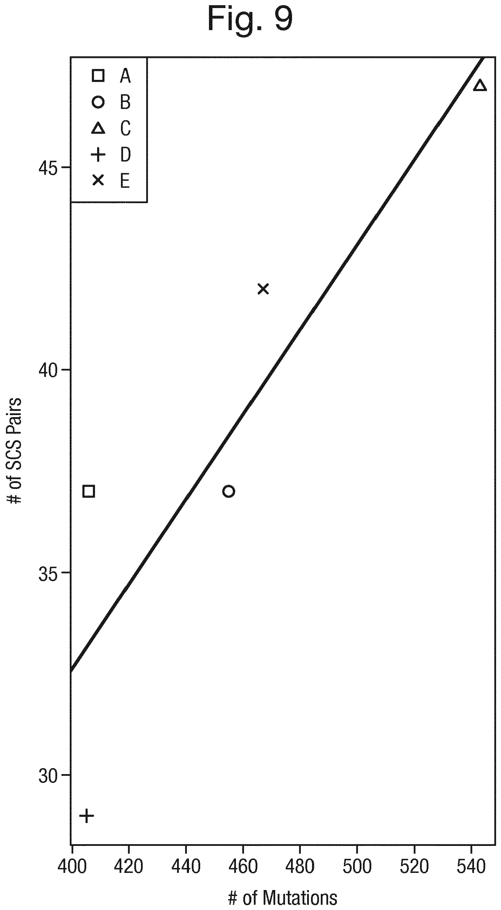

FIG. 9 is a graph illustrating the correlation between the synthetic cancer survival burden and the somatic mutation burden obtained by genomic nucleotide sequence analysis of five lung cancer cell lines, A (□), B (○), C (Δ), D (+), and E (x).

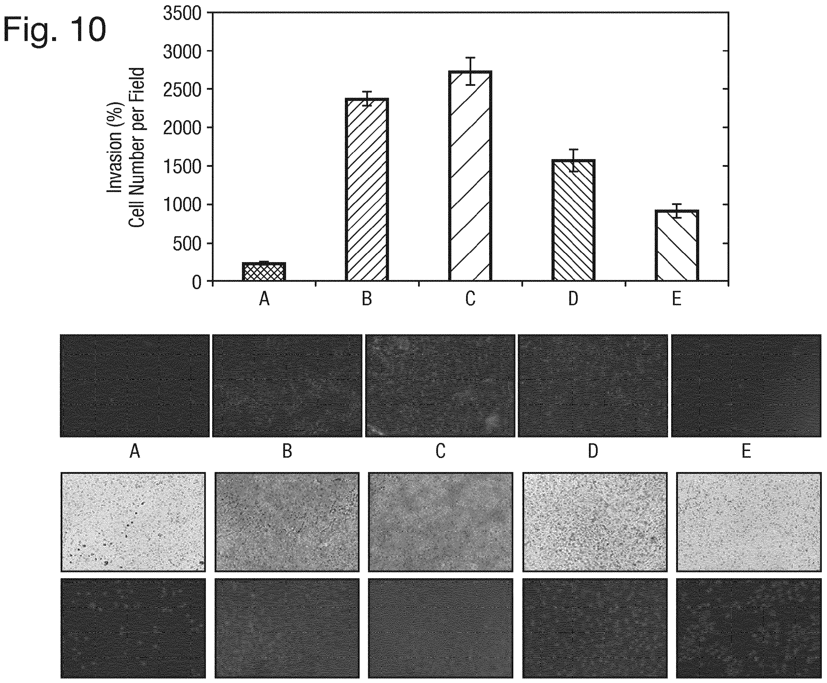

FIG. 10 is a bar graph of illustrating the results of identifying Matrigel invasive and metastatic ability which are obtained by three times experiments on five lung cancer cell lines, A (□), B (○), C (△), D (+), and E (x), using Matrigel invasion assay. The images of the three rows listed at the bottom of FIG. 10 are obtained by photographing the results of three Matrigel invasion assays for the five lung cancer cell lines.

[Best Mode]

The present invention departs from the conventionally known concept of synthetic lethality but is based on the concept of "synthetic cancer survival (SCS)," which is a combination of cancer patients whose survival rate is low when, among two genes of a specific patient, the functions of the two genes are normal or even when the function of either of the two genes is damaged and whose survival rate is high only when the functions of the two genes are damaged. The present invention is to provide a novel method of utilizing the concept for analyzing the interaction of genes, selecting customized anticancer therapeutic drugs, and predicting the prognosis of cancer patients.

An aspect of the present invention provides a method of providing information for customized anticancer therapeutic drug selection using a genomic nucleotide sequence variation of cancer patient, the method including: determining gene nucleotide sequence variant information of at least one gene belonging to a synthetic cancer survival pair of genes from the genomic nucleotide sequence information of the cancer patient; and selecting at least one candidate drug which inhibits at least one corresponding gene pairing with at least one variant gene belonging to the synthetic cancer survival pair of genes from the nucleotide sequence variant information.

The term "base sequence or nucleotide sequence" used in the present invention is a sequence in which bases, one of the constituents of a nucleotide which is the basic unit of nucleic acid DNA or RNA, are arranged in order.

The term "nucleotide sequence variant information" used in the present invention refers to, when the nucleotide sequence differs from the reference sequence to be compared, the region showing the difference and means information on substitution, addition or deletion of bases constituting gene's exon. Such substitution, addition or deletion of bases may be caused by various reasons. For example, it may be caused by structural difference such as chromosomal mutation, cleavage, deletion, duplication, inversion, and/or translocation.

The reference base sequence or reference genome is referred to as a reference nucleotide sequence or a standard nucleotide sequence which is used as a standard when the nucleotide sequences are compared.

Cancer genomic nucleotide sequence information used in the present invention can be determined using conventionally known nucleotide sequence analysis, which may, but not limited to, be provided by service providers such as BGI (Beijing Genome Institute), Knome, Macrogen, and DNALink that provide commercialized services.

The gene nucleotide sequence variant information included in the cancer genome nucleotide sequence in the present invention can be extracted using a variety of methods and can be obtained through a nucleotide sequence comparison and analysis using a nucleotide sequence comparison program with genomic nucleotide sequence of a reference group such as HG19, for example, ANNOVAR (Wang et al., Nucleic Acids Research, 2010; 38(16): e164), SVA (Sequence Variant Analyzer) (Ge et al., Bioinformatics. 2011; 27(14): 1998-2000), and BreakDancer (Chen et al., Nat Methods. 2009 Sep; 6(9): 677-81).

The gene nucleotide sequence variant information may be received/obtained through a computer system. In this aspect, the method of the present invention may further include receiving the gene mutation information with a computer system. The computer system used in the present invention may access to or include at least one database including a database in which information on anticancer therapeutic drugs applicable to cancer patients and information related to the gene inhibited by the drug can be retrieved or extracted.

The term "synthetic cancer survival (SCS)" used in the present invention refers to a phenomenon in which the combination of two or more variant genes included in cancer cells or cancer tissues leads to an improvement in the survival rate of the corresponding cancer patients, and each of the two or more variant genes does not cause an improvement in the survival rate of the corresponding cancer patients, but the combination of these two or more variant genes causes an improvement in the survival rate of the corresponding cancer patients. Term synthetic cancer survival used in the present invention does not refer only to the combination of two or more variant genes that cause the synthetic cancer survival occurs in the only single cancer cell. Even if the combination of two or more variant genes occurs in cancer cells different from each other, they are also called synthetic cancer survival when they occur in different cancer cells in the same cancer tissue to make the combination. In one embodiment of the present invention, a synthetic cancer survival gene is selected by analysis of cancer patient survival using genetic mutation information and survival information of cancer patients. In another embodiment of the present invention, a synthetic cancer survival gene is selected through the identification of invasive or metastatic ability and the genomic mutation analysis in the cancer cell line or cancer tissue.

The term "synthetic cancer survival pair of genes" used in the present invention means a gene pair with a combination of two or more variant genes included in a cancer cell or cancer tissue in which the gene pair induces an improvement in the survival rate of the corresponding cancer patients, and each of the two or more variant genes does not cause an improvement in the survival rate of the corresponding cancer patients, but the combination of these two or more variant genes causes an improvement in the survival rate of the corresponding cancer patients. Term synthetic cancer survival pair of genes used in the present invention does not refer only to the pair of genes that causes the synthetic cancer survival occurs in the only single cancer cell. Even if the combination of two or more variant genes occurs in cancer cells different from each other, they are also called synthetic cancer survival pair of genes when they occur in different cancer cells in the same cancer tissue to make the combination. When the two genes belonging to the synthetic cancer survival pair of genes are variant genes with a low gene deleteriousness score, the two genes are defined as constituting a synthetic cancer survival pair of genes. In addition, when one of the two genes belonging to the synthetic cancer survival pair of genes is a variant gene which has a low gene deleteriousness score, and the other is a corresponding gene which does not have a low gene deleteriousness score, if the corresponding gene is inhibited by a drug which inhibits the corresponding gene, the survival rate of the cancer patients can be expected to increase. In an embodiment of the present invention, the synthetic cancer survival pair of genes is selected through survival analysis using cancer genetic mutation and patient survival information, and specific examples thereof are shown in Table 2, but the scope of present invention is not limited thereto.

More specifically, in one embodiment of the present invention, a synthetic cancer survival pair of genes is selected through a cancer patient survival analysis using genetic mutation and survival information of cancer patients. The synthetic cancer survival pair of genes can be obtained using cancer cells or cancer tissues collected directly from cancer patients or using *in vitro* cancer cell line experiments or cancer tissue experiments. In this case, it may be presumed that the corresponding survival rate may be considered to be higher, as the invasive or metastatic ability is lower based on cancer cell's invasive or metastatic ability corresponding to the survival information of the cancer patients. It may be presumed that the corresponding survival rate may be considered to be lower, as the invasive or metastatic ability is higher. In other words, the synthetic cancer survival pair of genes according to the present invention may be obtained not only by clinical information of the patient group but also by cell, tissue, or animal experiments. In particular, in the case of cell, tissue, or animal experiments, the condition of a specific gene's function damaged can be implemented on an experimental basis through the experiment on inhibition of gene expression by mutagenesis, drug, RNA interference, and the like as well as naturally occurring genomic nucleotide sequence variants. Thus, it is possible to artificially induce a more diverse nucleotide sequence variant than a genomic nucleotide sequence variant of a cancer patient that can be observed in clinical practice or to perform a various experiment on inhibition of the corresponding gene's function, thereby obtaining more various synthetic cancer survival pairs of genes.

As such, synthetic cancer survival pairs of genes obtained by identifying the metastatic or invasive ability in cancer cell, tissue or animal experiments through nucleotide sequence variants that artificially cause mutations or a method of inhibiting the gene expression are included in the scope of the present invention as well as survival information and naturally occurring genomic nucleotide sequence variants of cancer patients.

The term "synthetic cancer survival" used in the present invention is a concept different from "synthetic lethality." The synthetic lethality is a phenomenon that a combination of nucleotide sequence variants of two or more genes causes cell death in which each of the nucleotide sequence variants of the two or more genes is a viable nucleotide sequence mutation/variant, but a combination of viable nucleotide sequence variants of the two or more genes causes cell death.

The synthetic lethality is a phenomenon that a combination of nucleotide sequence variants of two or more genes causes cell death. Being applied to cancers, the synthetic lethality is a phenomenon that a combination of nucleotide sequence variants of two or more genes causes the death of cancer cells. In the case of cancer, it is known that the cancer cell death may have some effect on the survival rate of the cancer patients, but its effect is limited, and the cancer metastasis has a more significant impact on the survival rate of cancer patients rather than cancer cell death. Further, the evaluation index of synthetic lethality is not the survival rate of cancer patients but cell death. The survival rate of synthetic cancer of the present invention is different from the synthetic lethality that leads to death of cancer cells and is referred to as a phenomenon that the gene variant of cancers induces a decrease in the ability to harm such as growth or metastasis ability on the corresponding cancer patients to result in an improvement in survival of the cancer patient. Thus, the synthetic cancer survival disclosed in the present invention is a different concept from the conventionally known synthetic lethality.

Further, in the case of the conventionally known synthetic lethality in which a combination of nucleotide sequence variants of two or more genes causes cell death, the corresponding cancer cell dies, so it can be observed *in vitro* but is difficult to be found in cancer tissues of patients in practice. On the other hand, the synthetic cancer survival is a phenomenon that occurs due to the combination of nucleotide sequence variants of two or more genes found in the cancer tissues of patients in practice and thus is a concept differentiating from the conventionally known synthetic lethality.

More specifically, as exemplified in Examples 1 to 3 of the present invention, the present inventors have found a large number of synthetic cancer survival pairs of genes in cancer tissues and cancer cell lines of various cancer types and have confirmed that the cancer tissues and cancer cell lines did not reach cell death but had lived still. From these results, it can be seen that the synthetic cancer survival, the concept of survival of cancer patients disclosed in the present invention as described above, is different from the synthetic lethality that refers to the concept of cell death.

Further, as exemplified in Examples 4 and 5 of the present invention, the present inventors have suggested a concept of synthetic cancer survival burden and have confirmed the positive linear correlation in which, as a patient has more synthetic cancer survival pairs of genes, the survival rate thereof has been higher. On the other hand, such a linear correlation is not discussed in the concept of synthetic lethality, and it is defined that the deleteriousness of even one synthetic lethality pair of genes leads to the irreversible death of the corresponding cell in the concept of synthetic lethality. Therefore, the concept of inducing more, greater or stronger deaths is not valid although two pairs, or three pairs, or more synthetic lethality pair of genes are found. Therefore, a concept such as "synthetic lethality burden' has not been established or proven. As it can be seen from the novel concept of the synthetic cancer survival burden, synthetic cancer survival and synthetic lethality are different concepts from each other.

In the present invention, the variant gene and the corresponding gene can be calculated based on the presence of a loss of function variant. Such functional loss mutations can include, but are not limited to, nonsense mutations, frameshift insertion and deletion, nonstop mutation and splice site mutation.

More specifically, the variant gene and the corresponding gene can be determined by the gene nucleotide sequence variant score included in each relevant gene.

The term "gene nucleotide sequence variant score" used in the present invention refers to, when a genomic nucleotide sequence variant is found in the exon region of a gene that encodes a protein, a score obtained by quantifying the degree of the meaningful change or damage on the structure and/or function of the relevant protein caused by the amino acid sequence variant (substitution, addition, or deletion), transcription regulatory variant, or the like of the protein encoded by the relevant gene, which are caused by this individual variant. The gene nucleotide sequence variant score can be calculated by considering the degree to which the structure or function of the protein changes depending on the degree of evolutionary conservation of the amino acid and physical properties of the modified amino acid on the genomic nucleotide sequence.

The gene nucleotide sequence variant score used in the method of calculating the gene deleteriousness score of the present invention can be calculated using a method known in the art. For example, the gene nucleotide sequence variant score may, but not be limited to, be produced from the gene nucleotide sequence variant information by applying an algorithm such as SIFT (Sorting Intolerant From Tolerant, Pauline C et al., Genome Res. 2001 May; 11(5): 863-874; Pauline C et al., Genome Res. 2002 March; 12(3): 436-446; Jing Hul et al., Genome Biol. 2012; 13(2): R9), PolyPhen, PolyPhen-2 (Polymorphism Phenotyping, Ramensky V et al., Nucleic Acids Res. 2002 September 1; 30(17): 3894-3900; Adzhubei IA et al., Nat Methods 7(4): 248-249 (2010)), MAPP (Eric A. et al., Multivariate Analysis of Protein Polymorphism, Genome Res. 2005; 15: 978-986), Logre (Log R Pfam E-value, Clifford R.J et al., Bioinformatics 2004; 20: 1006-1014), Mutation Assessor (Reva B et al., Genome Biol. 2007; 8: R232, http://mutationassessor.org/), Condel (Gonzalez-Perez A et al., The American Journal of Human Genetics 2011; 88: 440-449, http://bg.upf.edu/fannsdb/), GERP (Cooper et al., Genomic Evolutionary Rate Profiling, Genome Res. 2005; 15: 901-913, http://mendel.stanford.edu/SidowLab/downloads/gerp/), CADD (Combined Annotation-Dependent Depletion, http://cadd.gs.washington.edu/), MutationTaster, MutationTaster2 (Schwarz et al., MutationTaster2: mutation prediction for the deep-sequencing age. Nature Methods 2014; 11: 361-362, http://www.mutationtaster.org/), PROVEAN (Choi et al., PLoS One. 2012; 7(10): e46688), PMuit (Ferrer-Costa et al., Proteins 2004; 57(4): 811-819, http://mmb.pcb.ub.es/PMut/), CEO (Combinatorial Entropy Optimization, Reva et al., Genome Biol 2007; 8(11): R232), SNPeffect (Reumers et al., Bioinformatics. 2006; 22(17): 2183-2185, http://snpeffect.vib.be), fathmm (Shihab et al., Functional Analysis through Hidden Markov Models, Hum Mutat 2013; 34: 57-65, http://fathmm.biocompute.org.uk/), MSRV (Jiang, R. et al. Sequence-based prioritization of nonsynonymous single-nucleotide polymorphisms for the study of disease mutations. Am J Hum Genet 2007; 81: 346-360, http://msms.usc.edu/msrv/), Align-GVGD (Tavtigian, Sean V., et al. Comprehensive statistical study of 452 BRCA1 missense substitutions with classification of eight recurrent substitutions as neutral. Journal of medical genetics 2006: 295-305., http://agvgd.hci.utah.edu/), DANN (Quang, Daniel, Yifei Chen, and Xiaohui Xie. DANN: a deep learning approach for annotating the pathogenicity of genetic variants. Bioinformatics 2014: btu703., https://cbcl.ics.uci.edu/public_data/DANN/), Eigen (Ionita-Laza, Iuliana, et al. A spectral approach integrating functional genomic annotations for coding and noncoding variants. Nature genetics (2016): 214-220., http://www.columbia.edu/~ii2135/eigen.html), KGGSeq (Li MX, Gui HS, Kwan JS, Bao SY, Sham PC. A comprehensive framework for prioritizing variants in exome sequencing studies of Mendelian diseases. Nucleic Acids Res. 2012 Apr; 40(7): e53., http://grass.cgs.hku.hk/limx/kggseq/), LRT (Chun, Sung, and Justin C. Fay. Identification of deleterious mutations within three human genomes. Genome Res. 2009: 1553-1561., http://www.genetics.wustl.edu/jflab/lrt_query.html), MetaLR (Dong, Chengliang, et al. Comparison and integration of deleteriousness prediction methods for nonsynonymous SNVs in whole exome sequencing studies. Human molecular genetics 2015; 24(8): 2125-2137), MetaSVM (Dong, Chengliang, et al. Comparison and integration of deleteriousness prediction methods for nonsynonymous SNVs in whole exome sequencing studies. Human molecular genetics 2015; 24(8): 2125-2137), MutPred (Mort, Matthew, et al. MutPred Splice: machine learning-based prediction of exonic variants that disrupt splicing. Genome Biology 2014; (15)1: 1, http://www.mutdb.org/mutpredsplice/about.htm), PANTHER (Mi, Huaiyu, et al. The PANTHER database of protein families, subfamilies, functions and pathways. Nucleic Acids Research 2005; (33) suppl 1: D284-D288., http://www.pantherdb.org/tools/csnpScoreForm.jsp), Parepro (Tian, Jian, et al. Predicting the phenotypic effects of non-synonymous single nucleotide polymorphisms based on support vector machines. BMC bioinformatics 2007; 8.1, http://www.mobioinfor.cn/parepro/contact.htm), phastCons (Siepel, Adam, et al. Evolutionarily conserved elements in vertebrate, insect, worm, and yeast genomes. Genome Res. 2005; 915)8: 1034-1050, http://compgen.cshl.edu/phast/), PhD-SNP (Capriotti, E., Calabrese, R., Casadio, R. Predicting the insurgence of human genetic diseases associated to single point protein mutations with support vector machines and evolutionary information. Bioinformatics 2006; 22: 2729-2734., http://snps.biofold.org/phd-snp/), phyloP (Pollard, Katherine S., et al. Detection of nonneutral substitution rates on mammalian phylogenies._Genome Res. 2010; (20)1: 110-121., http://compgen.cshl.edu/phast/background.php), PON-P (Niroula, Abhishek, Siddhaling Urolagin, and Mauno Vihinen. PON-P2: prediction method for fast and reliable identification of harmful variants. PLoS One 2015; (10)2: e0117380., http://structure.bmc.lu.se/PON-P2/), SiPhy (Garber, Manuel, et al. Identifying novel constrained elements by exploiting biased substitution patterns. Bioinformatics 2009; (25)12: i54-i62, http://portals.broadinstitute.org/genome_bio/siphy/documentation.html), SNAP (Bromberg,Y. and Rost,B. SNAP: predict effect of non-synonymous polymorphisms on function. Nucleic Acids Res. 2007; 35: 3823-3835,w http://www.rostlab.org/services/SNAP), SNPs&GO (Remo Calabrese, Emidio Capriotti, Piero Fariselli, Pier Luigi Martelli, and Rita Casadio. Functional annotations improve the predictive score of human disease-related mutations in proteins. Human Mutatation 2009; 30: 1237-1244, http://snps.biofold.org/snps-and-go/), VEP (McLaren W, Pritchard B, Rios D, Chen Y, Flicek P and Cunningham F. Deriving the consequences of genomic variants with the Ensembl API and SNP Effect Predictor. Bioinformatics 2010; 26: 2069-70 http://www.ensembl.org/info/docs/tools/vep/), VEST (Carter H, Douville C, Stenson P, Cooper D, Karchin R Identifying Mendelian disease genes with the Variant Effect Scoring Tool BMC Genomics 2013; 14(Suppl 3): S3), SNAP2 (Yana Bromberg, Guy Yachdav, and Burkhard Rost. SNAP predicts effect of mutations on protein function. Bioinformatics 2008; 24: 2397-2398, http://www.rostlab.org/services/SNAP), CAROL (Lopes MC, Joyce C, Ritchie GR, John SL, Cunningham F et al. A combined functional annotation score for non-synonymous variants, http://www.sanger.ac.uk/science/tools/carol), PaPI (Limongelli, Ivan, Simone Marini, and Riccardo Bellazzi. PaPI: pseudo amino acid composition to score human protein-coding variants. BMC bioinformatics 2015; (16)1: 1, http://papi.unipv.it/), Grantham (Grantham, R. Amino acid difference formula to help explain protein evolution. Science 1974; (185)4154: 862-864, https://ionreporter.thermofisher.com/ionreporter/help/GUID-D9DFB21C-652D-4F95-8132-A0C442F65399.html), SInBaD (Lehmann, Kjong-Van, and Ting Chen. Exploring functional variant discovery in non-coding regions with SInBaD. Nucleic Acids Research 2013; (41)1: e7-e7, http://tingchenlab.cmb.usc.edu/sinbad/), VAAST (Hu, Hao, et al. VAAST 2.0: Improved variant classification and disease_]gene identification using a conservation_]controlled amino acid substitution matrix. Genetic epidemiology 2013; (37)6: 622-634, http://www.yandelllab.org/software/vaast.html), REVEL (Ioannidis, Nilah M., et al. REVEL: an Ensemble Method for Predicting the Pathogenicity of Rare Missense Variants._AGHG 2016, https://sites.google.com/site/revelgenomics/), CHASM (Carter H, Chen S, Isik L, Tyekucheva S, Velculescu VE, Kinzler KW, Vogelstein B, Karchin R Cancer-specific high-throughput annotation of somatic mutations: computational prediction of driver missense mutations Cancer Res 2009; 69(16): 6660-7, http://www.cravat.us), mCluster (Yue P, Forrest WF, Kaminker JS, Lohr S, Zhang Z, Cavet G: Inferring the functional effects of mutation through clusters of mutations in homologous proteins. Human mutation. 2010; 31(3): 264-271. 10.1002/humu.21194.), nsSNPAnayzer (Lei Bao, Mi Zhou, and Yan Cui nsSNPAnalyzer: identifying disease-associated nonsynonymous single nucleotide polymorphisms. Nucleic Acids Res 2005; 33: 480-482, http://snpanalyzer.uthsc.edu/), SAAPpred (Nouf S Al-Numair and Andrew C R Martin. The SAAP pipeline and database: tools to analyze the impact and predict the pathogenicity of mutations. BMC Genomics 2013; 14(3): 1-11, www.bioinf.org.uk/saap/dap/), HanSa (Acharya V. and Nagarajaram H.A. Hansa An automated method for discriminating disease and neutral human nsSNPs. Human Mutation 2012; 2: 332-337, hansa.cdfd.org.in:8080/), CanPredict (Kaminker,J.S. et al. CanPredict: a computational tool for predicting cancer-associated missense mutations. Nucleic Acids Res., 2007; 35: 595:598, http://pgws.nci.nih.gov/cgi-bin/GeneViewer.cgi_), FIS (Boris Reva, Yevgeniy Antipin, and Chris Sander. Predicting the functional impact of protein mutations: Application to cancer genomics. Nucleic Acids Res 2011; 39: e118-e118.), BONGO (Cheng T.M.K., Lu Y-E, Vendruscolo M., Lio P., Blundell T.L. Prediction by graph theoretic measures of structural effects in proteins arising from non-synonymous single nucleotide polymorphisms. PLoS Comp Biology 2008; (4)7: e1000135, http:// www.bongo.cl.cam.ac.uk/Bongo2/Bongo.htm) to gene nucleotide sequence variant included in each relevant gene. For example, when assigning a gene nucleotide sequence variant score using the SIFT score, a hypothesis that a variant having a SIFT score of 0.7 or greater does not cause a significant change in the function of the relevant gene is applied to utilize a filtering process in which the variant having 0.7 or greater is transformed into absence of a variant, and such a modification belongs to the scope of the present invention. For example, when assigning a gene nucleotide sequence variant score using the SIFT score, the score obtained by transforming the relevant SIFT score through an arbitrary function also belongs to the scope of the present invention.

The purpose of the algorithms as described above is to determine how much each gene nucleotide sequence variant affects the expression or function of the relevant protein and how much the effect damages the protein, or whether there is no other effect. These are basically common in that the amino acid sequence and the related changes of the protein encoded by the relevant gene, which are caused by the individual gene nucleotide sequence variant, are determined to evaluate the effect on the expression, structure and/or function of the relevant protein.

In one embodiment according to the present invention, a sorting intolerant from tolerant (SIFT) algorithm is used to calculate an individual gene nucleotide sequence variant score. In the case of the SIFT algorithm, for example, the gene nucleotide sequence variant information is input in a variant call format (VCF) file, and the degree to which each gene nucleotide sequence variant damages the relevant gene is scored. In the case of the SIFT algorithm, it is determined that the function of the relevant gene is more damaged due to the deleteriousness of the protein encoded by the relevant gene as the calculated score is closer to 0 and that the protein encoded by the relevant gene maintains normal function as the calculated score is closer to 1.

In the case of the other algorithm, PolyPhen-2, the higher the calculated score, the higher the degree of functional deleteriousness of the protein encoded by the relevant gene.

Recently, a study has been disclosed to compare with and put together the SIFT, Polyphen2, MAPP, Logre, and Mutation Assessors to suggest the Condel algorithm (Gonzalez-Peerez, A. & Lopez-Bigas, N. Improving the assessment of the outcome of nonsynonymous SNVs with a consensus deleteriousness score, Condel. The American Journal of Human Genetics, 2011: 88(4): 440-449). In this study, the five algorithms are compared using HumVar and HumDiv (Adzhubei, IA et al., A method and server for predicting damaging missense mutations. Nature Methods, 2010; 7 (4): 248-249), which are conventionally known sets of data related to the gene nucleotide sequence variant which damages a protein and the gene nucleotide sequence variant which has less effect on a protein.

As a result, a 97.9% gene nucleotide sequence variant causing protein deleteriousness of HumVar and a 97.3% gene nucleotide sequence variant having less effect of protein thereof are equally detected in at least three algorithms among the five algorithms. A 99.7% gene nucleotide sequence variant causing protein deleteriousness of HumDiv and a 98.8% gene nucleotide sequence variant having less effect on protein thereof are equally detected in at least three algorithms among the five algorithms. Further, the five algorithms and the respective algorithms are integrated to produce to draw receiver operating curve (ROC) showing the accuracy of the results thereof for HumVar and HumDiv. As a result, it is confirmed that area under receiver operating curve (AUC) has considerably high level (69% to 88.2%) conformity. In other words, the various algorithms are significantly correlated with the calculated gene nucleotide sequence variant scores although the calculation methods are different. Therefore, the calculation of the gene nucleotide sequence variant scores by applying the algorithms or methods utilizing the algorithms is within the scope of the present invention regardless of the different algorithms. When the gene nucleotide sequence variant occurs in the exon region of a gene encoding a protein, it may directly affect the expression, structure and/or function of the protein. Thus, the gene nucleotide sequence variant information can be related to the degree of protein function deleteriousness. In this aspect, the method of the present invention includes the concept of calculating a "gene deleteriousness score" based on gene nucleotide sequence variant scores. More specifically, the variant gene and the corresponding gene can be determined by the gene deleteriousness score calculated from the gene nucleotide sequence variant score calculated by applying the algorithm as described above to the gene nucleotide sequence variant included in each relevant gene.

In the present invention, the variant gene and the corresponding gene can be determined by the gene deleteriousness score calculated as the mean value of each gene nucleotide sequence variant score when there are two or more gene nucleotide sequence variants included in each relevant gene.

The term "gene deleteriousness score (GDS)" used in the present invention means the score calculated by incorporating the gene nucleotide sequence variant scores when at least two significant nucleotide sequence variants are found in the gene region encoding one protein, and the one protein has at least two gene nucleotide sequence variant scores. If there is one significant nucleotide sequence variant in the gene region encoding the protein, the gene deleteriousness score is calculated the same as the relevant gene nucleotide sequence variant score. In this regard, when there are at least two gene nucleotide sequence variants encoding the protein, the gene deleteriousness score is calculated as the mean value of the gene nucleotide sequence variant scores calculated for each variant. The mean value may, but be not limited to, be calculated by, for example, a geometric mean, an arithmetic mean, a harmonic mean, an arithmetic-geometric mean, an arithmetic-harmonic mean, a geometric-harmonic mean, a Pythagorean mean, a quartile mean, a quadratic mean, a truncated mean, a winsorized mean, a weighted mean, a weighted geometric mean, a weighted arithmetic mean, a weighted harmonic mean, a function mean, a power mean, a generalized f-mean, percentile, maximum, minimum, mode, median, central range, measures of central tendency, simple product or weighted product, or a function of the above calculated values.

In one embodiment according to the present invention, the gene deleteriousness score is calculated by the following Equation 1. However, the following Equation 1 can be modified in various ways, so the present invention is not limited thereto.

[Equation 1]

$$S_g(v_{1,...,}v_n) = \left(\frac{1}{n}\sum_{i=1}^{n}v_i^p\right)^{\frac{1}{p}}$$

In Equation 1, $S_g$ is a gene deleteriousness score of the protein encoded by gene $g$, $n$ is the number of nucleotide sequence variants to be analyzed among the nucleotide sequence variants of the gene $g$, $v_i$ is a nucleotide sequence variant score of $i$-th nucleotide sequence variant to be analyzed, and $p$ is a non-zero real number.

In Equation 1, when the value of $p$ is 1, the arithmetic mean is obtained. When the value of $p$ is -1, the harmonic mean is obtained. When the value of $p$ is a limit close to 0, the geometric mean is obtained.

In another embodiment according to the present invention, the gene deleteriousness score is calculated by the following Equation 2.

[Equation 2]

$$S_g(v_{1,...,}v_n) = \left(\prod_{i=1}^{n}v_i^{w_i}\right)^{1/\sum_{i=1}^{n}w_i}$$

In Equation 2, $S_g$ is a gene deleteriousness score of the protein encoded by gene $g$, $n$ is the number of nucleotide sequence variants to be analyzed among the nucleotide sequence variants of the gene $g$, $vi$ is a gene nucleotide sequence variant score of $i$-th nucleotide sequence variant to be analyzed, and $w_i$ is a weight given to the gene nucleotide sequence variant score $vi$ of the $i$-th nucleotide sequence variant.

When all the weights $w_i$ have the same value, the gene deleteriousness score $S_g$ is a geometric mean value of the gene nucleotide sequence variant score $vi$. The weight may be given in consideration of the type of the relevant protein, the pharmacokinetic or pharmacodynamic classification of the relevant protein, the pharmacokinetic parameter of the relevant drug enzyme protein, and the population group or the distribution by race.

The nucleotide sequence variant scores and gene deleteriousness scores according to the present invention are disclosed in Korean Patent Application No. 10-2014-0107916 and PCT International Application No. PCT/KR2014/007685, and the disclosures thereof are incorporated herein by reference in its entirety.

The method according to the present invention may further include determining a priority of drugs to be applied to cancer patients using the synthetic cancer survival pair of genes information or determining whether to use the drugs to be applied to cancer patients using the synthetic cancer survival pair of genes information.

The method according to the present invention may further include dividing into at least two subgroups based on a significantbiological marker by cancer types and then conducting a survival analysis using the genomic mutant information and patient survival information in each subgroup to select the synthetic cancer survival pair of genes.

The biological marker is related to diagnosis, treatment, and prognosis associated with cancers, which is a concept that includes all markers known in the art. For example, known markers for each cancer type can be used without limitation, including, for example, microsatellite instability (MSI), known as a biological marker essential for diagnosis, treatment, and prognosis of colorectal cancer.In the present invention, the selection of the candidate drug may be performed by calculating the number of at least one variant gene pairing with at least one corresponding gene belonging to the synthetic cancer survival pair of genes selected from the genomic nucleotide sequence information of the cancer patient to determine the priority or combination of candidate drugs based on the calculated number.

In another aspect, the present invention provides a system for selecting a customized anticancer therapeutic drug using genomic nucleotide sequence variant information of a cancer patient, the system including: a database in which information related to an anticancer therapeutic drug to be applied to cancer patients and a gene inhibited by the drug is searched or extracted; a communication unit accessible to the database; a cancer genomic nucleotide sequence analyzer; a drug selection information provider; and a display, in which the cancer genomic nucleotide sequence analyzer includes: a variant gene selector selecting at least one variant gene belonging to a synthetic cancer survival pair of genes; and a corresponding gene selector selecting at least one corresponding gene pairing with the relevant at least one variant gene constituting the synthetic cancer survival pair of genes, and in which the drug selection information provider provides anticancer therapeutic drug selection information for inhibiting the relevant at least one corresponding gene.

The system according to the present invention may further include a user interface accessing to the database capable of searching and extracting information related to an anticancer therapeutic drug to be applied to a cancer patient and a gene inhibited by the drug and extracting the related information to provide the customized drug selection information to a user.

In the system according to the present invention, the database or the server including the access information of the database, the calculated information, and the user interface device connected thereto can be used in association with each other.

In the system according to the present invention, a user interface or a terminal may request a customized anticancer therapeutic drug selection process and receive and/or store the result thereof. The user interface or the terminal may include memory such as a smartphone, a personal computer (PC), a tablet PC, a personal digital assistant (PDA), and a web pad and may be equipped with a microprocessor to be constituted as a terminal having a mobile communication function with operation ability.

In the system according to the present invention, the server is a means for providing access to the database and is configured to be able to exchange various information by being connected to a user interface or a terminal through a communication unit. In this regard, the communication unit may be performed in the same hardware, and further the communication may be carried out by a local area network (LAN), a metropolitan area network (MAN), a wide area network (WAN), Internet, 2G, 3G, and 4G mobile communication network, Wi-Fi, WiBro, and the like. The communication method is not limited to wired or wireless, and any communication method may be used. The database can be installed directly on the server or can be connected directly to a variety of life sciences databases accessible via the Internet and the like for its purpose.

The method according to the present invention may be implemented in hardware, firmware, or software, or a combination thereof. When implemented in software, the storage medium includes any medium that stores or transfers the same in a form readable by a device, such as a computer. For example, the computer-readable medium may include read only memory (ROM), random access memory (RAM), magnetic disk storage medium, optical storage medium, flash memory device, other electrical, optical, or acoustic signal transmission medium, and the like.

In this aspect, the present invention provides a computer-readable medium including an executable module for executing a processor performing an operation including: selecting a synthetic cancer survival pair of genes from genomic nucleotide sequence information of a cancer patient; and selecting at least one candidate drug that inhibits at least one corresponding gene pairing with at least one variant gene belonging to the synthetic cancer survival pair of genes.

The method and system for selecting a customized drug using genomic mutant information and survival information of cancer patients according to the present invention are techniques which can select an anticancer therapeutic drug with excellent therapeutic effect and prognosis by an individual to provide highly reliable relevant information quickly and simply through the nucleotide sequence variant analysis of the synthetic cancer survival pair of genes derived from the genomic mutant information and survival information of cancer patients.

Using the method and system according to the present invention, at least one variant gene belonging to a gene pair inducing synthetic cancer survival is selected, and at least one corresponding gene pairing with the relevant variant gene to constitute the synthetic cancer survival pair of genes is selected, thereby selecting at least one anticancer therapeutic drug that inhibits the corresponding gene so that it is possible to select a customized anticancer agent by an individual from several comparative drugs. By predicting drug effects or the risk of side effects in advance, it is possible to determine the priority, optimum combination, or use of anticancer agents. Further, the combination of at least one variant genes found in a plurality of patients having the relevant cancer type by specific cancer type is selected from the combinations of variant genes belonging to the synthetic cancer survival pair of genes, thereby selecting a combination of at least one anticancer therapeutic drug, which is predicted to have a good prognosis and therapeutic effect in a large number of patients of the relevant cancer type in general, which is independent of the genome nucleotide sequence analysis results of individual patients. This is a technique that can be used for the development and clinical application of combination chemotherapy specified by cancer types, which is highly reliable to provide relevant information quickly and simply.

Further, the method and system according to the present invention can be used to predict cancer prognosis by analyzing the frequency and distribution of nucleotide sequence variants of a synthetic cancer survival pair of genes for each individual. The frequency and distribution of nucleotide sequence variant for each individual of a somatic mutation and a synthetic cancer survival pair of genes are analyzed and thus are used to predict the prognosis of cancer. In addition, the frequency and distribution analysis of individual nucleotide sequence variants of a synthetic cancer survival pair of genes and somatic mutation can be efficiently used to predict therapeutic drug response.

In still another aspect, the present invention provides a method of providing information for predicting prognosis of a cancer patient, the method including calculating the number of at least one gene belonging to the synthetic cancer survival pair of genes from nucleotide sequence information of a cancer patient genome.

The method may include calculating the number of at least one gene belonging to the synthetic cancer survival pair of genes and the number of somatic mutation gene from nucleotide sequence information of a cancer patient genome.

In one embodiment of the present invention, it is confirmed that the survival rate of cancer patients is statistically significantly higher as the number of synthetic cancer survival pairs of genes is increased. Thus, the survival prognosis of the relevant cancer patient can be effectively predicted by confirming the synthetic cancer survival burden represented by the number of synthetic cancer survival pair of genes of the cancer patient through genomic analysis of the cancer patient.

In yet another aspect, the present invention provides a system for selecting a customized anticancer therapeutic drug using genomic nucleotide sequence variant information of a cancer patient, the system including: a database in which information related to an anticancer therapeutic drug to be applied to cancer patients and a gene inhibited by the drug is searched or extracted; a communication unit accessible to the database; a cancer genomic nucleotide sequence analyzer; a drug selection information provider; and a display, in which the cancer genomic nucleotide sequence analyzer includes: a variant gene pair selector selecting at least one variant gene belonging to a synthetic cancer survival pair of genes; and a corresponding gene selector selecting at least one corresponding gene pairing with the relevant at least one variant gene constituting the synthetic cancer survival pair of genes, and in which the drug selection information provider provides drug selection information for increasing the number of synthetic cancer survival pairs of genes of the cancer patient.

In one embodiment of the present invention, it is confirmed that when a drug selected by applying a customized drug selection method is administered to a patient, the therapeutic response to the drug can also be predicted by analyzing the number of synthetic cancer survival pairs of genes which are increased due to genes inhibited by the relevant therapeutic drug. More specifically, it is confirmed that the relevant therapeutic response can be predicted according to the degree of the number of the synthetic cancer survival pair of genes of the relevant patient increased by the therapeutic drug, and conversely, a drug having excellent improvement in the therapeutic response can be selected as a customized therapeutic drug.

In yet another aspect, the present invention provides a computer-readable medium including an executable module for executing the processor performing an operation including: selecting a synthetic cancer survival pair of genes from genomic nucleotide sequence information of a cancer patient; and selecting a candidate drug that increases the number of synthetic cancer survival pairs of genes among at least one candidate drug that inhibits at least one corresponding gene pairing with at least one variant gene belonging to the synthetic cancer survival pair of genes.

Since the computer readable medium used in the present invention has already been described above, the description thereof is excluded in order to avoid excessive duplication.

[Mode for Invention]

Hereinafter, preferred Examples are provided to help understand the present invention. However, the following Examples are provided only for the easier understanding of the present invention, and the scope of the present invention is not limited by Examples.

**Example 1. Detection of synthetic cancer survival pair of genes by cancer type and method of selecting customized drug using the same**

1-1. Target data selection

The data for the analysis was downloaded from TCGA data portal on March 4, 2015. The data includes level 2 somatic mutation data of 5,618 persons and level 2 clinical data of 6,838 persons. The level 2 somatic mutation data has been stored in a mutation annotation format (maf). For the analysis, mutation positions and mutation classification were applied. The mutations are classified into 'Missense mutation,' 'Nonsense mutation,' 'Frameshift indel,' 'In frame indel,' 'splice site mutation; Silent mutation,' 'Intron,' 'UTR' and 'Intergenic.' The level 2 clinical data includes various clinical variables according to cancer type, and the variables actually used in the Cox proportional hazards model were examined by a professional pathologist.

**1-2. Data processing and analysis data configuration**

First, data from patients without information for the Cox proportional hazards model were excluded from the clinical data. Next, after identifying patients with other malignancies or metastases and patients who received radiotherapy, pharmaco, and ablation adjuvant therapy, it is considered that these factors were strong disturbances in the prognosis of the patients, and thus the data of the patients were removed. Further, data from patients without mutation data were excluded. More specifically, for the mutation data, first, the synonymous mutations were excluded, and then, the genes indicated as 'Unknown' in the data as a gene without the HGNC symbol were excluded. Finally, data from patients without clinical information were excluded, and the further analysis used data from 4,844 persons.

As a result of data processing, clinical data and somatic mutation data for 4,884 persons were obtained from 20 cancer types. The obtained data have both types of data and have all the clinical variable data required for the Cox proportional hazards model to be used for the further analysis.

1-3. Gene deleteriousness score

In this Example, a gene deleteriousness score (GDS) was defined to quantify the degree of deleteriousness of a gene. The gene deleteriousness score was calculated by considering the number and type of mutations in the relevant gene and was defined to have a value between 0 and 1 point. The gene deleteriousness score was defined to mean that the smaller the score, the worse the functional structural deleteriousness of the relevant gene. For example, if a gene has a loss of function (LoF) variant such as nonsense mutation, frameshift insertion and deletion, nonstop mutation and splice site mutation, the gene deleteriousness score of the relevant gene is 0 point. If a gene has no LoF variant, the gene deleteriousness score of the relevant gene is determined as the geometric mean of the SIFT score of mutations with a SIFT score of 0.7 points or less among all non-synonymous mutations present in the relevant gene. In this regard, when the SIFT score is 0 point, this is substituted with 10e-8 points in order to avoid the case where the denominator is zero. The filtering criterion of the SIFT score of 0.7 is an arbitrary filtering criterion applied in the case of this Example, and various filtering criteria can be applied according to the analysis purpose. Further, the variant score of 10e-8 points given to prevent the denominator from being 0 is an arbitrary criterion applied in the case of this Example, and various criteria can be applied according to the analysis purpose. In this Example, the SIFT algorithm used to calculate the gene deleteriousness score (*See* Equation 3 below) is also an arbitrary algorithm applied in the case of this Example, and various algorithms can be applied according to the analysis purpose.

$$[\text{Equation 3}]$$

$$GDS_g \begin{cases} 0, if\ gene\ has\ LoF\ mutation \\ \left(\prod_{k=1}^{n} SIFT(v_k)\right)^{\frac{1}{n}}, else \end{cases}$$

1-4. Setting of distribution and analysis threshold of gene deleteriousness score

The gene deleteriousness scores of all genes having at least one non-synonymous mutation in each cancer type were calculated based on the analysis data classified in Example 1-2. A gene having no non-synonymous mutation was assigned gene deleteriousness score of 1 point.

As a result, although many somatic mutations occur in cancer cells, it is not common that somatic mutations occur in whole genes. Thus, it was confirmed that most genes had a gene deleteriousness score of 1 point. In addition to 1 point, gene deleteriousness scores of many genes showing somatic mutation were distributed at 0 points. In this Example, a gene deleteriousness score of 0.3 points was used as a criterion (analysis threshold value) to divide genes into two groups: genes with gene function deleteriousness at moderate degree or more or genes without the same so that they were used for further analysis.

1-5. Detection of synthetic cancer survival pair of genes by cancer types and establishment of synthetic cancer survival gene network by cancer types

Cox proportional hazards model was used to conduct survival analysis in order to detect synthetic cancer survival (SCS) in genomic data of cancer patients. Cox proportional hazards model can correct disturbances of clinical variables. Patient group by each cancer type was divided into 4 groups for all gene pairs: both-deleteriousness group in which both genes had gene deleteriousness scores of 0.3 or less, two only-deleteriousness groups in which one of two genes had gene deleteriousness scores of 0.3 or less and the other did not have such score, and none-deleteriousness group in which both genes had gene deleteriousness scores of 0.3 or more.

In case of Cox proportional hazards model based on maximum likelihood, which is commonly used, 'convergence' problem occurs when the patient death case is zero. Thus, the Cox proportional hazards model utilizing the penalized likelihood was used in this Example to avoid this problem. Survival analysis was conducted using the 'coxphf package of R Statistical Package version 3.2.0. Further, it was added to Cox model to correct disturbance of clinical variables by each cancer type. General clinical variables such as age and gender and other clinical variables reviewed by pathology specialists and used in previous studies were added thereto.

FIG. 1 illustrates the respective survival curves in which the skin cutaneous melanoma patients were divided into four groups according to the somatic mutation status of the DNAH2 gene and the XIRP2 gene pair: one both-deleteriousness group, two only-deleteriousness groups and one none-deleteriousness group. In this regard, survival analysis results are shown along with the survival curves of the 4 groups. As illustrated in FIG. 1, it can be seen that the DNAH2 gene and the XIRP2 gene were in a relationship of a synthetic cancer survival pair of genes. Namely, in the DNAH2 and XIRP2 pair, the cancer survival rate of the only-deleteriousness group in which only DNAH2 gene deleteriousness score was low (blue line) or only XIRP2 gene deleteriousness score was low (yellow line) was not significantly different compared to that of the none-deleteriousness group in which both genes deleteriousness scores were not low (green line). However, it was confirmed that the survival rate of cancer patients of the both-deleteriousness group in which both DNAH2 and XIRP2 gene deleteriousness scores were low were statistically significantly higher than other three groups ($p < 0.05$ and $HR > 1.0$). Therefore, it was confirmed that the DNAH2 gene and the XIRP2 gene pair which shows somatic mutation in the skin cutaneous melanoma satisfied the criteria of the synthetic cancer survival pair of genes of the skin cutaneous melanoma as defined above.

Further, FIG. 2 illustrates a synthetic cancer survival gene network consisting of synthetic cancer survival pairs of genes obtained for the respective cancer types in five cancer types (lung adenocarcinoma, skin cutaneous melanoma, lung squamous cell carcinoma, head and neck squamous cell carcinoma and kidney renal clear cell carcinoma). The synthetic cancer survival pair of genes of lung adenocarcinoma (LUAD) is represented by red connection line, the synthetic cancer survival pair of genes of skin cutaneous melanoma (SKCM) is represented by yellow connection line, the synthetic cancer survival pair of genes of lung squamous cell carcinoma (LUSC) is represented by blue connection line, the synthetic cancer survival pair of genes of head and neck squamous cell carcinoma (HNSC) is represented by brown connection line, and the synthetic cancer survival pair of genes of kidney renal clear cell carcinoma (KIRP) is represented by purple connection line. As illustrated in FIG. 2, it can be confirmed that a variety of synthetic cancer survival (SCS) pairs of genes exist for each cancer type, and a detailed description thereof is disclosed in Example 2 below.

In this Example, various synthetic cancer survival pairs of genes were obtained through analysis of cancer genomic mutation information of actual cancer patients. However, this method is one of various applicable methods, and the present invention is not limited thereto. For example, gene variants can be induced in a cell line or an animal experiment environment in various ways to analyze variant genes that are not observed in actual cancer patients, thereby obtaining a synthetic cancer survival pair of genes and constituting a synthetic cancer survival genes network. In particular, a synthetic cancer survival pair of genes can be obtained using various experimental methods for identifying the cancer cell metastatic ability including Invasion Assay as exemplified in Example 5 and FIGS. 9 and 10.

1-6. Method of selecting customized drug using analysis of synthetic cancer survival pair of genes by cancer types

The following experiment was carried out to discover effectively and efficiently the synthetic cancer survival pairs of genes through the method and system of survival analysis and genomic mutation of cancer patients according to the present invention and to describe the method of performing a customized drug selection using the same.

The distribution of somatic mutations of one lung adenocarcinoma patient is overlaid on the network of synthetic cancer survival pair of genes in FIG. 3. The nodes and connection lines in FIG. 3 refer to the network of synthetic cancer survival pair of genes obtained by analyzing genomic sequencing data of the lung adenocarcinoma. In this regard, the node refers to each gene, and a pair of genes connected by a connection line refers to a synthetic cancer survival pair of genes of lung adenocarcinoma. The red colored gene node refers to a gene in which a somatic mutation is found, which pairs with the corresponding gene to constitute a synthetic cancer survival pair of genes in the relevant cancer patients. The yellow colored gene node refers to a gene with a somatic mutation having low gene deleteriousness score in which there is no corresponding gene with a somatic mutation showing a low gene deleteriousness score among genes paired with the relevant gene constituting a synthetic cancer survival pair of genes so that the gene did not constitute the synthetic cancer survival pair of genes. The gray colored gene node refers to a gene that does not have a somatic mutation having a low gene deleteriousness score in the relevant cancer patient.

Therefore, FIG. 3 illustrates how several synthetic cancer survival pairs of genes are formed with other genes by inhibiting at least one gene selected by considering synthetic cancer survival gene network information among gray colored genes as at least blocker for the relevant gene. For example, when cancer cells of a lung adenocarcinoma patient illustrated in FIG. 3 is treated with XIRP2 blocker, it can be predicted that the gene pairs with genes such as RYR2, LPA, and FAT4 to constitute a plurality of synthetic cancer survival pairs of genes, thereby improving the survival rate of the lung adenocarcinoma patients. Further, it is known that although RYR3 is blocked in cancer cells of a lung adenocarcinoma patient, the gene may pair with several genes to constitute a synthetic cancer survival pair of genes in which RYR3 can be blocked by calcium channel blockers such as Dandrolene. Recently, specific genes can be blocked through the development of antibody drugs, so target genes for new drug development can be also selected through an analysis of synthetic gene pairs by the present invention. According to one study such as Zhang et al., Proc Natl Acad Sci U S A. 2011 Aug 16; 108 (33): 13653-13658, it is disclosed that prognoses of ovarian cancer varies depending on the single nucleotide polymorphism of the binding site of micro-RNA miR-367 which inhibits RYR3. It is not yet clear whether these findings are due to the effect of blocking RYR3, a key participant gene of the synthetic cancer survival pair of genes, the result of the present invention. However, it can be presumed that the probability of the academic prospect is high by showing the difference in the prognoses through the relationship between synthetic cancer survival genes which are the result of the present invention. New drugs should be developed not only in terms of their effectiveness but also in terms of safety such as side effects, and this Example is based on the analysis of next generation sequencing data of cancer patient genomic information to utilize characteristics of synthetic cancer survival pair of genes found in the present invention, thereby providing useful information for the selection and development of customized drugs for cancer patients.

**Example 2. Prediction of distribution and prognosis of synthetic cancer survival pair of genes by cancer type**

As shown in the above Example 1, the analysis of the synthetic cancer survival pair of genes indicated that 436 synthetic cancer survival pairs of genes were selected from 5 cancer types, and the results are shown in Table 1 ($p < 0.05$ and $HR > 1$). The selection criteria of the synthetic cancer survival pair of genes used in this Example are strictly applied. It is clear that various conditions can be combined for detecting a synthetic cancer survival pair of genes. Synthetic cancer survival pairs of genes by cancer types were selected by applying a strict criterion in which there was a statistically significant difference in comparison between the both-deleteriousness and none-deleteriousness groups as illustrated in Example 1, and there was a statistically significant difference in the comparison of each only-deleteriousness group and both-deleteriousness group, but there was no statistically significant difference in three comparisons of each only-deleteriousness group and none-deleteriousness group.

[Table 1]

| Tumor Type | Num. of SCS pairs | Clinical variables used in cox model |
|---|---|---|
| LUAD | 287 | Age, Gender, Pathologic T/N stage |
| SKCM | 137 | Age, Pathologic T/N stage, Marginal status, ER/PR/HER2 status |
| LUSC | 6 | Age, Grade, Clinical Stage |
| HNSC | 5 | Age, Gender, Pathologic T/N stage, vascular/lymphovascular invasion status, Anatomic neoplasm subdivision |
| KIRP | 1 | Age, Gender, Karnofsky score |
| Total | 436 | |

As shown in Table 1, in particular, a large number of synthetic cancer survival pairs of genes were selected from lung adenocarcinoma (LUAC) and skin cutaneous melanoma (SKCM), and 436 synthetic cancer survival pairs of genes selected in this Example consisted of 281 genes more specifically. XIRP2, RYR3, and the like were genes belonging to the most numerous synthetic cancer survival pairs of genes.

The determination criteria of this Example were applied to obtain 436 synthetic cancer survival pairs of genes for each of the five cancer types, which are shown in Table 2.

[Table 2]

| Tumor Type | SCS gene pairs | |
|---|---|---|
| HNSC | CDKN2A | PRKDC |
| HNSC | COL11A1 | CSMD3 |
| HNSC | CSMD3 | NSD1 |
| HNSC | HLA-B | NOTCH1 |
| HNSC | MUC16 | ZNF99 |
| KIRP | MUC16 | TTN |
| LUAD | A2ML1 | ASPM |

(continued)

| Tumor Type | SCS gene pairs | |
|---|---|---|
| LUAD | A2ML1 | C6 |
| LUAD | A2ML1 | FAM5C |
| LUAD | A2ML1 | GRIN2B |
| LUAD | A2ML1 | PAPPA2 |
| LUAD | A2ML1 | UNC13C |
| LUAD | A2ML1 | XIRP2 |
| LUAD | A2ML1 | ZEB1 |
| LUAD | ABCA6 | FLG |
| LUAD | ABCA6 | ZFHX4 |
| LUAD | ABCB5 | C1orf173 |
| LUAD | ABCB5 | C7orf58 |
| LUAD | ABCB5 | DUSP27 |
| LUAD | ABCB5 | TTN |
| LUAD | ACACA | PIK3C2B |
| LUAD | ACACA | ZFHX4 |
| LUAD | ADCY10 | CARD8 |
| LUAD | ADCY10 | CSMD3 |
| LUAD | ADCY10 | XIRP2 |
| LUAD | AFF2 | DST |
| LUAD | AFF2 | SPTA1 |
| LUAD | AKAP6 | C6 |
| LUAD | AKAP6 | KCNB2 |
| LUAD | AKAP6 | MYO3B |
| LUAD | AKAP6 | RYR2 |
| LUAD | AKAP6 | RYR3 |
| LUAD | AKAP6 | SMARCA4 |
| LUAD | AKAP6 | THSD7A |
| LUAD | AKAP6 | TNN |
| LUAD | AKAP6 | XIRP2 |
| LUAD | AKAP6 | ZEB1 |
| LUAD | AMER1 | GRIN2B |
| LUAD | AMER1 | XIRP2 |
| LUAD | ASPM | RYR3 |
| LUAD | ASPM | SCN10A |
| LUAD | ASPM | XIRP2 |
| LUAD | ATP2B3 | KIF21B |
| LUAD | ATP2B3 | RYR3 |
| LUAD | C12orf63 | MUC16 |

(continued)

| Tumor Type | SCS gene pairs | |
|---|---|---|
| LUAD | C18orf34 | PAPPA2 |
| LUAD | C1orf173 | ROS1 |
| LUAD | C6 | KCNB2 |
| LUAD | C6 | MUC2 |
| LUAD | C6 | SLC1A3 |
| LUAD | C6 | THSD7A |
| LUAD | C6 | TNN |
| LUAD | C6 | UNC13C |
| LUAD | C6 | XIRP2 |
| LUAD | C7orf58 | HCN1 |
| LUAD | C7orf58 | MYOM2 |
| LUAD | C7orf58 | ROS1 |
| LUAD | C7orf58 | XIRP2 |
| LUAD | CACNA1E | FAT4 |
| LUAD | CACNA1E | FOLH1 |
| LUAD | CACNA1E | GRM7 |
| LUAD | CACNA1E | KIF21B |
| LUAD | CACNA1E | LILRA1 |
| LUAD | CACNA1E | SLC12A1 |
| LUAD | CACNA1E | SMARCA4 |
| LUAD | CACNA1E | ZNF99 |
| LUAD | CARD8 | CSMD3 |
| LUAD | CCDC178 | PAPPA2 |
| LUAD | CDH23 | PSG8 |
| LUAD | CDH23 | ZFHX4 |
| LUAD | CDH7 | GPR158 |
| LUAD | CENPE | PAPPA2 |
| LUAD | CENPE | PCDHAC2 |
| LUAD | CENPE | XIRP2 |
| LUAD | CENPE | ZNF804A |
| LUAD | CENPF | RYR3 |
| LUAD | CENPF | XIRP2 |
| LUAD | CHD8 | COL11A1 |
| LUAD | CMYA5 | XIRP2 |
| LUAD | CNKSR2 | RYR2 |
| LUAD | CNTN5 | COL7A1 |
| LUAD | CNTNAP2 | HYDIN |
| LUAD | COL11A1 | FER1L6 |

(continued)

| Tumor Type | SCS gene pairs | |
|---|---|---|
| LUAD | COL11A1 | FRAS1 |
| LUAD | COL11A1 | ITPR2 |
| LUAD | COL11A1 | KLK1 |
| LUAD | COL11A1 | TSHZ3 |
| LUAD | COL4A4 | RYR3 |
| LUAD | COL4A5 | PCDHGC5 |
| LUAD | COL7A1 | TNN |
| LUAD | COL7A1 | XIRP2 |
| LUAD | CPED1 | CSMD1 |
| LUAD | CPED1 | DUSP27 |
| LUAD | CPED1 | HCN1 |
| LUAD | CPED1 | MYOM2 |
| LUAD | CPED1 | ROS1 |
| LUAD | CPED1 | SYNE1 |
| LUAD | CPED1 | TNXB |
| LUAD | CPS1 | DCHS2 |
| LUAD | CPS1 | GRM7 |
| LUAD | CPS1 | HCN1 |
| LUAD | CPS1 | LRRIQ1 |
| LUAD | CPS1 | PCDHAC2 |
| LUAD | CPS1 | RYR2 |
| LUAD | CPS1 | SYNE1 |
| LUAD | CPS1 | UNC13C |
| LUAD | CREBBP | RYR3 |
| LUAD | CREBBP | TNN |
| LUAD | CSMD1 | FCGBP |
| LUAD | CSMD1 | GRM7 |
| LUAD | CSMD1 | MYOM2 |
| LUAD | CSMD1 | OR2W3 |
| LUAD | CSMD1 | PDE3A |
| LUAD | CSMD1 | PLXNA2 |
| LUAD | CSMD1 | SALL1 |
| LUAD | CSMD1 | THSD7A |
| LUAD | CSMD1 | TRPA1 |
| LUAD | CSMD3 | DYSF |
| LUAD | CSMD3 | ITGAV |
| LUAD | CSMD3 | MYO7A |
| LUAD | CSMD3 | SCN3A |

(continued)

| Tumor Type | SCS gene pairs | |
|---|---|---|
| LUAD | CYP11B2 | XIRP2 |
| LUAD | DCDC1 | FAM5C |
| LUAD | DCDC1 | XIRP2 |
| LUAD | DCDC5 | FAM5C |
| LUAD | DCHS2 | RYR3 |
| LUAD | DMXL1 | RYR3 |
| LUAD | DSCAM | KCNB2 |
| LUAD | DSCAM | RYR2 |
| LUAD | DSCAM | UNC13C |
| LUAD | DSCAML1 | USH2A |
| LUAD | DST | NID2 |
| LUAD | DUSP27 | FAT4 |
| LUAD | DUSP27 | GRIN2B |
| LUAD | DUSP27 | HTR1E |
| LUAD | DUSP27 | PEG3 |
| LUAD | DUSP27 | RYR3 |
| LUAD | DYSF | KMT2A |
| LUAD | EGFLAM | RYR2 |
| LUAD | F8 | FAT4 |
| LUAD | F8 | KRAS |
| LUAD | FAM123B | GRIN2B |
| LUAD | FAM47B | RYR3 |
| LUAD | FAM47B | TNN |
| LUAD | FAM47C | GRM1 |
| LUAD | FAM47C | MYO18B |
| LUAD | FAM47C | TUBA3C |
| LUAD | FAM47C | ZNF804A |
| LUAD | FAM5C | FAT4 |
| LUAD | FAM5C | KIF21B |
| LUAD | FAM5C | OR2W3 |
| LUAD | FAM5C | SCN9A |
| LUAD | FAM5C | SMARCA4 |
| LUAD | FAM5C | SYNE1 |
| LUAD | FAM5C | TNN |
| LUAD | FAM5C | ZEB1 |
| LUAD | FAT1 | FLG |
| LUAD | FAT2 | RYR3 |
| LUAD | FAT3 | FOLH1 |

(continued)

| Tumor Type | SCS gene pairs | |
| --- | --- | --- |
| LUAD | FAT3 | KIF21B |
| LUAD | FAT3 | OR5AS1 |
| LUAD | FAT4 | GRM1 |
| LUAD | FAT4 | HCN1 |
| LUAD | FAT4 | NLGN4X |
| LUAD | FAT4 | PDZRN3 |
| LUAD | FAT4 | RYR3 |
| LUAD | FAT4 | XIRP2 |
| LUAD | FAT4 | ZNF804A |
| LUAD | FCGBP | TTN |
| LUAD | FOLH1 | HCN1 |
| LUAD | FOLH1 | UNC13C |
| LUAD | FOLH1 | UNC79 |
| LUAD | FOLH1 | XIRP2 |
| LUAD | FOLH1 | ZNF804A |
| LUAD | GRIN2B | KCNB2 |
| LUAD | GRIN2B | MYO18B |
| LUAD | GRIN2B | PRKCB |
| LUAD | GRIN2B | ROS1 |
| LUAD | GRIN2B | ZNF804A |
| LUAD | GRM1 | OR2G2 |
| LUAD | GRM7 | HCN1 |
| LUAD | GRM7 | RYR3 |
| LUAD | GRM7 | TTN |
| LUAD | GRM7 | ZNF804A |
| LUAD | HCN1 | MYO18B |
| LUAD | HCN1 | PTPRB |
| LUAD | HCN1 | RYR3 |
| LUAD | HCN1 | SYNE1 |
| LUAD | HCN1 | XIRP2 |
| LUAD | HCN1 | ZEB1 |
| LUAD | HFM1 | RYR2 |
| LUAD | HTR1E | TNN |
| LUAD | HTR1E | UNC13C |
| LUAD | INSRR | MUC16 |
| LUAD | ITPR2 | TSHZ3 |
| LUAD | KCNB2 | MYO3B |
| LUAD | KCNB2 | SLC1A3 |

(continued)

| Tumor Type | SCS gene pairs | |
| --- | --- | --- |
| LUAD | KCNB2 | TNN |
| LUAD | KCNB2 | UNC13C |
| LUAD | KCNB2 | XIRP2 |
| LUAD | KCNH7 | XIRP2 |
| LUAD | KIF21B | MUC2 |
| LUAD | KIF21B | PAPPA2 |
| LUAD | KIF21B | PLXNA2 |
| LUAD | KIF5A | XIRP2 |
| LUAD | KLK1 | RYR2 |
| LUAD | KLK1 | TSHZ3 |
| LUAD | LAMA1 | LPA |
| LUAD | LAMA1 | RYR3 |
| LUAD | LAMA1 | XIRP2 |
| LUAD | LILRA1 | NBPF10 |
| LUAD | LPA | MYO18B |
| LUAD | LPA | PDZRN3 |
| LUAD | LPA | PTPRB |
| LUAD | LPA | RYR3 |
| LUAD | LPA | SLC12A1 |
| LUAD | LPA | TNN |
| LUAD | LRBA | MYOM2 |
| LUAD | LRRIQ1 | RYR3 |
| LUAD | LRRIQ1 | TNN |
| LUAD | LRRIQ1 | ZEB1 |
| LUAD | LRRIQ3 | ZNF804A |
| LUAD | LTBP1 | OTOGL |
| LUAD | MLL2 | XIRP2 |
| LUAD | MMRN1 | MYO18B |
| LUAD | MMRN1 | PDZRN3 |
| LUAD | MMRN1 | XIRP2 |
| LUAD | MUC2 | PCDH11X |
| LUAD | MUC2 | XIRP2 |
| LUAD | MYO18B | PHF14 |
| LUAD | MYO18B | RYR3 |
| LUAD | MYO3B | PEG3 |
| LUAD | MYO3B | SLC1A3 |
| LUAD | MYO3B | UNC13C |
| LUAD | MYO7A | RYR2 |

(continued)

| Tumor Type | SCS gene pairs | |
|---|---|---|
| LUAD | MYOM2 | PAPPA2 |
| LUAD | MYOM2 | PCDHAC2 |
| LUAD | MYOM2 | RYR2 |
| LUAD | MYOM2 | ZNF804A |
| LUAD | MYT1L | RYR3 |
| LUAD | MYT1L | TNN |
| LUAD | MYT1L | TRPA1 |
| LUAD | MYT1L | UNC79 |
| LUAD | MYT1L | XIRP2 |
| LUAD | NBPF10 | RYR2 |
| LUAD | NBPF10 | RYR3 |
| LUAD | NBPF10 | TTN |
| LUAD | NOL4 | TTN |
| LUAD | NOL4 | XIRP2 |
| LUAD | OR2T33 | TTN |
| LUAD | OR2W3 | PDE3A |
| LUAD | OR4A15 | ZNF536 |
| LUAD | OR5D13 | RYR3 |
| LUAD | PAPPA2 | SLC1A3 |
| LUAD | PAPPA2 | UNC13C |
| LUAD | PCDHAC2 | PDE3A |
| LUAD | PCDHAC2 | SCN9A |
| LUAD | PCDHAC2 | SLC26A7 |
| LUAD | PDE3A | ZNF804A |
| LUAD | PDZRN3 | RYR2 |
| LUAD | PDZRN3 | RYR3 |
| LUAD | PDZRN3 | TNN |
| LUAD | PEG3 | SYCP2 |
| LUAD | PHACTR1 | SLC6A18 |
| LUAD | RIMS2 | USH1C |
| LUAD | ROBO4 | RYR3 |
| LUAD | ROS1 | XIRP2 |
| LUAD | RYR2 | THSD7A |
| LUAD | RYR2 | XIRP2 |
| LUAD | RYR3 | SLC1A3 |
| LUAD | RYR3 | SMARCA4 |
| LUAD | RYR3 | UNC13C |
| LUAD | SCN10A | SYNE1 |

(continued)

| Tumor Type | SCS gene pairs | |
|---|---|---|
| LUAD | SCN10A | XIRP2 |
| LUAD | SLC6A18 | TCF20 |
| LUAD | SLC6A18 | UHRF1BP1L |
| LUAD | SMARCA4 | XIRP2 |
| LUAD | SMARCA4 | ZNF804A |
| LUAD | SPTA1 | TNRC6A |
| LUAD | SVEP1 | TNN |
| LUAD | SVEP1 | ZNF99 |
| LUAD | SYNE1 | XIRP2 |
| LUAD | TCF20 | ZFHX4 |
| LUAD | TIAM1 | USH2A |
| LUAD | TNN | UNC13C |
| LUAD | TNN | YLPM1 |
| LUAD | TNRC6A | ZFHX4 |
| LUAD | TRPA1 | XIRP2 |
| LUAD | TUBA3C | XIRP2 |
| LUAD | UNC13C | XIRP2 |
| LUAD | XIRP2 | ZNF99 |
| LUAD | YLPM1 | ZEB1 |
| LUAD | ZNF804A | ZNF99 |
| LUSC | CDH10 | FAM135B |
| LUSC | CSMD3 | PCDHAC2 |
| LUSC | CSMD3 | PEG3 |
| LUSC | CSMD3 | TTN |
| LUSC | LRP1B | SCN1A |
| LUSC | PCDHAC2 | TP53 |
| SKCM | ABCA4 | BPTF |
| SKCM | ADAM28 | PDE1A |
| SKCM | ADAMTSL1 | FAT3 |
| SKCM | ADAMTSL3 | TP53 |
| SKCM | ADD2 | ARMC4 |
| SKCM | ANK3 | CDH6 |
| SKCM | ANK3 | LAMA1 |
| SKCM | ANKRD30B | MYH1 |
| SKCM | ARID2 | FREM1 |
| SKCM | ASPM | CLCN1 |
| SKCM | ASPM | MLL3 |
| SKCM | ASPM | MYH6 |

(continued)

| Tumor Type | SCS gene pairs | |
|---|---|---|
| SKCM | ASTN1 | COL4A2 |
| SKCM | ASTN1 | FREM1 |
| SKCM | ASTN1 | GHR |
| SKCM | ASTN1 | ODZ1 |
| SKCM | ASTN1 | TENM1 |
| SKCM | ASTN1 | ZAN |
| SKCM | ATP1A3 | FAT3 |
| SKCM | ATP1A3 | GRID2 |
| SKCM | ATP1A3 | SCN5A |
| SKCM | BCLAF1 | FLG |
| SKCM | BCLAF1 | LRRC4C |
| SKCM | BCLAF1 | NBEA |
| SKCM | BCLAF1 | UGT2A3 |
| SKCM | BRAF | GALNT14 |
| SKCM | BRAF | MYO5B |
| SKCM | BRAF | NOTCH4 |
| SKCM | BRAF | TNN |
| SKCM | C12orf51 | UNC13C |
| SKCM | C7orf58 | PAPPA2 |
| SKCM | CACNA1C | CCDC88C |
| SKCM | CACNA1C | NBEA |
| SKCM | CACNA1C | PREX2 |
| SKCM | CACNA1C | RIMBP2 |
| SKCM | CACNA1C | SCN7A |
| SKCM | CACNA1E | NES |
| SKCM | CATSPERB | CDH6 |
| SKCM | CATSPERB | COL4A4 |
| SKCM | CCDC88C | COL5A3 |
| SKCM | CDH6 | FLG |
| SKCM | CDH6 | RYR1 |
| SKCM | CDH6 | TRPC4 |
| SKCM | CDHR2 | KCNB2 |
| SKCM | CES1 | PREX2 |
| SKCM | CLCN1 | KMT2C |
| SKCM | CLCN1 | MLL3 |
| SKCM | CLCN1 | SYNE1 |
| SKCM | CNTN5 | PROL1 |
| SKCM | COL21A1 | FLG |

(continued)

| Tumor Type | SCS gene pairs | |
|---|---|---|
| SKCM | COL21A1 | PROL1 |
| SKCM | COL21A1 | SACS |
| SKCM | COL2A1 | UGT2A3 |
| SKCM | COL4A4 | YLPM1 |
| SKCM | COL5A3 | DSCAM |
| SKCM | COL5A3 | GRID2 |
| SKCM | COL5A3 | KIF4B |
| SKCM | COL5A3 | PTPRN2 |
| SKCM | COL7A1 | NBEA |
| SKCM | CPED1 | PAPPA2 |
| SKCM | DAB1 | ST6GAL2 |
| SKCM | DNAH5 | KCNQ5 |
| SKCM | DNAH8 | GHR |
| SKCM | DPYD | OR2G3 |
| SKCM | DSCAM | MED12L |
| SKCM | DUSP27 | SPAG17 |
| SKCM | ENAM | FLG |
| SKCM | ENAM | PXDNL |
| SKCM | FAM5C | KDR |
| SKCM | FAM5C | XIRP2 |
| SKCM | FAT3 | GRM7 |
| SKCM | FAT3 | MAGEC1 |
| SKCM | FAT3 | NBEA |
| SKCM | FLG | GRID2 |
| SKCM | FLG | KIAA2022 |
| SKCM | FLG | LCT |
| SKCM | FLG | MYH2 |
| SKCM | FLG | NES |
| SKCM | FLG | PCDHA9 |
| SKCM | FLG | PREX2 |
| SKCM | FREM1 | PDE1A |
| SKCM | FRY | MYH2 |
| SKCM | GFRAL | PEG3 |
| SKCM | GHR | PAPPA2 |
| SKCM | GHR | TPTE |
| SKCM | GK2 | KCNB2 |
| SKCM | GK2 | MYH4 |
| SKCM | GPR98 | SLC14A2 |

(continued)

| Tumor Type | SCS gene pairs | |
|---|---|---|
| SKCM | GRID2 | PDE1A |
| SKCM | GRID2 | SERPINI2 |
| SKCM | GRIK3 | MYH7 |
| SKCM | GRM7 | PCDHA9 |
| SKCM | HECTD4 | UNC13C |
| SKCM | HSPG2 | RIMBP2 |
| SKCM | HYDIN | KIAA2022 |
| SKCM | HYDIN | TP53 |
| SKCM | KIF4B | PTPRN2 |
| SKCM | KRT1 | PAPPA2 |
| SKCM | LAMA1 | NPAP1 |
| SKCM | LCT | MYO18B |
| SKCM | LCT | RYR1 |
| SKCM | LCT | SACS |
| SKCM | LCT | SCN10A |
| SKCM | LRP1B | SACS |
| SKCM | LRRC4C | OR2G3 |
| SKCM | LRRC7 | PDE1A |
| SKCM | MLL3 | TRHDE |
| SKCM | MROH2B | TRPV5 |
| SKCM | MYH7 | UGT2A3 |
| SKCM | NES | PEG3 |
| SKCM | NES | PTPRB |
| SKCM | NES | SPHKAP |
| SKCM | NLRP5 | PAPPA2 |
| SKCM | NRAS | SCN5A |
| SKCM | OR1N2 | XIRP2 |
| SKCM | OR2G3 | PXDNL |
| SKCM | OR4K2 | TP53 |
| SKCM | OR51B5 | RYR1 |
| SKCM | OTOGL | PEG3 |
| SKCM | OTOGL | RGPD4 |
| SKCM | PADI3 | PKHD1L1 |
| SKCM | PAPPA2 | PEG3 |
| SKCM | PCDHA9 | PPP1R3A |
| SKCM | PCLO | UGT2A3 |
| SKCM | PDE1A | TEX15 |
| SKCM | PDE1C | PREX2 |

(continued)

| Tumor Type | SCS gene pairs | |
|---|---|---|
| SKCM | PDZD2 | SPEN |
| SKCM | PREX2 | UGT2A3 |
| SKCM | PTCHD2 | PTPRT |
| SKCM | PXDNL | TRHDE |
| SKCM | PXDNL | ZAN |
| SKCM | PXDNL | ZFPM2 |
| SKCM | RIMBP2 | SCN10A |
| SKCM | SACS | SCN5A |
| SKCM | SHANK2 | TP53 |
| SKCM | SI | SLC15A2 |
| SKCM | UGT2A3 | USH2A |

When both of two genes included in a synthetic cancer survival pair of genes are variant genes with low gene deleteriousness scores, the relevant two genes are defined as constituting a synthetic cancer survival pair of genes. When one of two genes included in a synthetic cancer survival pair of genes is a variant gene with a low gene deleteriousness score, and the other is a corresponding gene with no low gene deleteriousness score, it is predicted that a drug inhibiting the relevant corresponding gene is used to increase the survival rate of the relevant cancer patient.

FIG. 2 illustrates a gene network in multiple graphs, which consists of synthetic cancer survival pairs of genes shown in Table 2. In this regard, each node refers to a gene, and a pair of genes connected to each other by a connection line refers to a synthetic cancer survival pair of genes.

Further, FIG. 4 is a bar graph showing the frequency of variant genes having a gene deleteriousness score of 0.3 or less in the lung adenocarcinoma patient group. FIG. 5 illustrates the frequency in which variant genes included in a synthetic cancer survival pair of genes detected in lung adenocarcinoma were found in the lung adenocarcinoma patient.

As illustrated in FIGS. 4 and 5, it can be seen that the XIRP2 and RYR3 genes constitute a synthetic cancer survival pair of genes in many patients. On the other hand, it can be seen that in the case of the TTN gene, the number of patients with low gene deleteriousness scores of the TTN gene was high, but the number of patients with the TTN gene constituting the synthetic cancer survival pair of genes was relatively small. In other words, conventional studies have focused on the somatic mutation frequency of cancer genes, but it is not easy to predict the prognosis and therapeutic response of cancer patients simply by mutation analysis of individual genes, and analysis of gene pairs and gene network as the present invention significantly contribute to the prediction of prognosis and treatment response of cancer patients.

**Example 3. Prediction of cancer survival and prognosis using synthetic cancer survival burden by cancer type**

Effect of the number of synthetic cancer survival pair of genes on the prognosis and survival rate of cancer patients was analyzed. For example, results from 341 lung adenocarcinoma patients (LUAD) and 181 skin cutaneous melanoma patients (SKCM), respectively, are illustrated in FIGS. 6 and 7.

First, 341 lung adenocarcinoma patients were divided into three groups: 149 persons who did not have any synthetic cancer survival pair of genes, 122 persons who had 1 or more to less than 10 synthetic cancer survival pairs of genes, and 70 persons who had 10 or more synthetic cancer survival pairs of genes, and survival analysis was conducted using Cox proportional hazards model. As a result, it was confirmed that as illustrated in FIG. 6, the survival rate of 70 persons having the most numerous synthetic cancer survival pair of genes (10 or more) was the highest, the survival rate of 122 persons having more than 1 to less than 10 was the median, and the survival rate of 149 persons with no synthetic cancer survival pair of genes was the lowest. Therefore, it was confirmed that the survival rate of the lung adenocarcinoma patients was statistically significantly higher as the number of synthetic cancer survival pairs of genes was higher.

Next, 181 skin cutaneous melanoma patients were divided into three groups: 88 persons who did not have any synthetic cancer survival pair of genes, 47 persons who had 1 or more to less than 5 synthetic cancer survival pairs of genes, and 46 persons who had 5 or more synthetic cancer survival pairs of genes, and survival analysis was conducted using Cox proportional hazards model. As a result, it was confirmed that as illustrated in FIG. 7, it was confirmed that the survival rate of the skin cutaneous melanoma patients was statistically significantly higher as the number of synthetic cancer survival pairs of genes was higher.

Through the experiments as described above, it was confirmed that the synthetic cancer survival burden represented by the number of synthetic cancer survival pairs of genes of cancer patients through the genomic analysis of cancer patients was confirmed so that the survival prognosis of cancer patients can be efficiently predicted.

**Example 4. Prediction of cancer survival and prognosis using somatic mutation burden and synthetic cancer survival burden by cancer type**

Analysis of the cancer survival rate utilizing the number of synthetic cancer survival pair of genes found in the cancer patients disclosed in Example 3 is significantly important in the medical field. It is why these are different from one generally known that as non-synonymous somatic mutations in cancer cells are more, the cancer patients have a poor prognosis.

More specifically, the number of synthetic cancer survival pairs of genes and the frequency of non-synonymous somatic mutations are shown in a log-log graph (*See* FIG. 8). As illustrated in FIG. 8, the number of synthetic cancer survival pairs of genes is directly proportional to the frequency of non-synonymous somatic mutations in both lung adenocarcinoma and skin cutaneous melanoma. Therefore, according to the conventional general view that as the somatic mutations are more, the prognosis becomes worse, it may be determined that as the number of cancer survival pairs of genes directly proportional to the somatic mutation burden is greater, it is more likely that the prognosis becomes worse. However, the results of Example 3 show that the more the number of synthetic cancer survival pairs of genes, the better the prognosis. In other words, as described in Example 3, in the case of a patient having a large number of synthetic cancer survival pair of genes, it can be seen that the somatic mutation thereof is likely to increase as well, but variants of the synthetic cancer survival pair of genes, which is a specific type of somatic mutation, are more so that the prognosis may be better instead.

The inverse correlation of the effect of somatic mutation burden and synthetic cancer survival burden by cancer types on the cancer patients' prognosis can be clearly confirmed in the survival analysis graphs of the respective groups illustrated in the bottom of FIGS. 6 and 7. More specifically, the three survival analysis graphs at the bottom of FIG. 6 indicate that, as a result of conducting survival analysis by dividing 341 lung adenocarcinoma patients into three groups according to the number of retained cancer survival pairs of genes, patients with higher somatic mutation burden (74 persons, 61 persons, and 35 persons, respectively) represented by red color had statistically significantly worse prognoses than patients with lower somatic mutation burden (75 persons, 61 persons, and 35 persons, respectively) represented by sky blue color in all three groups.

Further, the three survival analysis graphs at the bottom of FIG. 7 indicate that, as a result of conducting survival analysis by dividing 181 skin cutaneous melanoma patients into three groups according to the number of retained cancer survival pairs of genes, patients with higher somatic mutation burden (44 persons, 23 persons, and 23 persons, respectively) represented by red color had statistically significantly worse prognoses than patients with lower somatic mutation burden (44 persons, 24 persons, and 23 persons, respectively) represented by sky blue color in all three groups.

These results are consistent with the conventional theory that if the number of synthetic cancer survival pairs of genes is corrected, the prognosis is worse as the number of somatic mutations increases. Conversely, even when the number of somatic mutations is large through the analysis results illustrated in FIGS. 6 and 7, it can be understood that when the number of mutations is corrected, the synthetic cancer survival pair of genes burden is a significant predictor of cancer prognosis.

Overall, the concept of the analysis of the synthetic cancer survival pair of genes presented in the present invention is different from that of the known somatic mutation analysis. In other words, it may be predicted that if somatic mutation burdens are the same, the prognosis of the relevant cancer patient is better as the synthetic cancer survival burden is larger, and if the cancer burdens are the same, the prognosis of the relevant cancer patient is better as the somatic mutation burden is smaller. For prediction of the prognosis of cancer patients, this phenomenon may be functionalized to provide information on synthetic cancer survival burden and somatic mutation burden obtained through cancer genomic analysis.

Further, as described in Example 1, it can be seen that when a drug selected by applying a customized drug selection method of cancer patients is administered to a patient, the therapeutic response to the drug is also predicted through analysis of the number of synthetic cancer survival pairs of genes which is increased by genes inhibited by the drug. In other words, the therapeutic response can be predicted according to the degree of increase in the number of synthetic cancer survival pairs of genes of the relevant patient by the therapeutic drug, and conversely, a drug having an improvement in the therapeutic response can be selected as a customized therapeutic drug.

**Example 5: Prediction of cancer cell metastatic ability using synthetic cancer survival burden and somatic mutation burden by cancer type**

Cancer patients die due to cancer metastasis rather than from cancer. It is why cancer tissue itself can be removed or controlled by topical treatments such as radiation therapy, but the treatment of metastatic cancer is very difficult, and the metastatic cells cause various harms. In other words, it can be presumed that the prognosis of cancer becomes better as the number of synthetic cancer survival pairs of genes, which is the result of the present invention, which is related to a decrease in the metastatic ability of the relevant cancer cells by the synthetic cancer survival pair of genes. Currently, cell invasion assay is one of the methods to identify the metastatic ability of cancer cells. For example, the Matrigel invasion assay provided by Corning Inc. is a gelatin-type protein mixture secreted by Engelbreth-Holm-Swarm (EHS) mouse sarcoma cells, which is an experimental method that can quantitatively evaluate how much cancer cells have the ability to invade this Matrigel.

Whole exome sequencing (WXS) and Matrigel invasion assay were conducted on five lung cancer cell lines (A, B, C, D, and E) in order to analyze the effect of synthetic cancer survival pairs of genes on cancer metastases. The experiments were conducted twice to be verified. In the first experiment, experimental conditions were controlled in which the final concentration of Matrigel was 300 $\mu$g/ml, the incubation time was 24 hours, and the number of cells used was about 75000 per well. The experiments were repeated twice in the second experiment, experimental conditions were controlled in which the final concentration of Matrigel was 300 $\mu$g/ml, the incubation time was 42 hours, and the number of cells used was about 75000 per well. The experiment was carried out three times in total. WXS used illnumina HiSeq 2000 System and Hg19 version of Human Reference Genome.

FIG. 9 illustrates the distribution of somatic mutation burden and synthetic cancer survival burden of the five cell lines. FIG. 9 illustrates that the number of synthetic cancer survival pairs of genes increases in direct proportion to the number of somatic mutations as described in Example 4. FIG. 10 illustrates a bar graph of Matrigel invasive or metastatic ability for each cell line as a result of the Matrigel invasion assay. In other words, the greater the number of cells invaded per field, the greater the invasive or metastatic ability of the relevant cancer cells, which indicates high cancer metastatic ability. Therefore, it was determined that C, B, D, E, and A cell lines in order had a high ability for cancer metastasis.

Using the distribution of somatic mutation burden and synthetic cancer survival burden illustrated in FIG. 9, it was predicted that the cancer metastatic ability of A whose synthetic cancer survival burden was higher was lower in comparison of D and A whose somatic mutation burdens were just over 400, and this was confirmed as expected in the bar graph of FIG. 10. Further, it was predicted that the cancer metastatic ability of E whose synthetic cancer survival burden was higher was lower in comparison of B and E whose somatic mutation burdens were around 460, and this was confirmed as expected in the bar graph of FIG. 10. Further, it was predicted that the cancer metastatic ability of B whose somatic mutation burden was higher was higher in comparison of B and A whose synthetic cancer survival burdens were 37, and this was confirmed as expected in the bar graph of FIG. 10. Therefore, it was confirmed that the cancer cell metastatic ability could be evaluated by analysis of synthetic cancer survival pair of genes, which is the result of the present invention. Matrigel invasion assay was conducted to identify invasive ability or metastatic ability of cancer cells or tissues in this Example, but the present invention is not limited thereto. For example, in order to evaluate the invasive ability or the metastatic ability of cancer cells or tissues, there is a method of more directly identifying invasive ability or the metastatic ability of cancer cells or tissues by transplanting cancer cells or tissues into experimental animals whose immune competence is restricted. The scope of the present invention includes the customized drug selection method in which synthetic cancer survival pair of genes is found by these various methods of identifying invasive ability or the metastatic ability of cancer cells or tissues, and the synthetic cancer survival phenomena are utilized.

**Example 6. Usefulness of analysis of synthetic cancer survival pair of genes according to classification of subgroup of cancer using biological marker**

This Example illustrates a method in which cancer types to be analyzed are divided into subgroups using specific biological markers, then synthetic cancer survival pairs of genes are detected, and customized drug selection and prognosis are predicted. In other words, this Example is divided not only by the conventional clinical and pathological cancer classification systems, but also by subgroup according to biological markers related to major diagnosis, treatment, and prognosis in the analysis of synthetic cancer survival by cancer types exemplified in Examples 1 to 4. Thus, the analysis of synthetic cancer survival can be conducted more accurately. This Example indicates that the analysis of synthetic cancer survival using such biological markers falls within the scope of the present invention.

For example, microsatellite instability (MSI) is known to be a very critical biological marker for the diagnosis, treatment, and prognosis of colon adenocarcinoma. This Example shows that the synthetic cancer survival analysis is conducted by dividing patient groups according to the MSI status in colon adenocarcinoma, which derives the result of the synthetic cancer survival analysis corresponding to Examples 1 to 4 as described above and further results in more useful and stable precision analysis results.

Colon adenocarcinoma (COAD) data were downloaded from the National Cancer Institute's Genomic Data Commons (NCI GDC) data portal in the U.S. on July 11, 2016 and TCGA Data Portal on March 21, 2016. Among the data, NCI GDC data includes somatic mutation data for 433 persons, and TCGA data includes microsatellite instability (MSI) data for 458 persons and clinical data for 459 persons. The somatic mutation data was in the form of a variant call format (VCF) file, which was sorted according to the human standard genome GRCh38 standard, and the variant was determined by MuTect2. The level 2 clinical data included various clinical variables, and pathologists selected the variables used in the Cox proportional hazards model. The MSI data were classified into 'MSS,' 'MSI-L,' and 'MSI-H' according to the MSI status of respective patients. This Example was analyzed in which MSI-L and MSI-H groups were classified into MSI-positive group, and MSS group was classified into MSI-negative group.

Data were excluded from patients who did not have the information for applying the Cox proportional hazards model and patients with other malignant tumor positive, or metastatic positive, radiotherapy, drug, or ablation adjuvant therapy. Further, patients without somatic mutation data and MSI data were excluded. After annotating the mutation with variant annotation tool (VAT) and excluding the synonymous mutation, the data of the gene without the HGNC symbol were excluded. Finally, data from patients without clinical information and MSI data were excluded. Lastly, 427 colon adenocarcinoma patients were used for analysis.

First, for total 427 colon adenocarcinoma patients, the method as described in Examples 1 and 2 was performed to attempt to find synthetic cancer survival pairs of genes, but no significant cancer survival pair of genes was found. In colon adenocarcinoma, the number of somatic mutations and prognosis varied according to MSI status, thereby dividing into 151 persons in MSI-positive group and 276 persons in MSI-negative group. Colon adenocarcinoma patients were divided into two groups according to MSI status, and then 14 significant synthetic cancer survival pairs of genes (p < 0.05 and HR > 1) were detected in the MSI-positive group (MSI-L and MSI-H). However, none of the synthetic cancer survival pairs of genes were found in MSI-negative group with low somatic mutation burden. Table 3 shows the synthetic cancer survival pair of genes of colon adenocarcinoma detected in the MSI-positive group.

[Table 3]

| 14 synthetic cancer survival pairs of genes obtained in MSI-positive group of colon adenocarcinoma by applying the criteria of this Example | |
|---|---|
| Gene A | Gene B |
| BRAF | COL6A3 |
| | PTPRS |
| | SYNE1 |
| OBSCN | KMT2B |
| | PCLO |
| | PIK3CA |
| PIK3CA | DCHS1 |
| | HMCN1 |
| DNAH1 | DYNC2H1 |
| | SPEG |
| COL6A3 | MYO7A |
| DYNC2H1 | KIAA1109 |
| HMCN1 | PCSK5 |
| SYNE1 | PCDH10 |

As shown in Table 3, 14 synthetic cancer survival pairs of genes were constituted with 17 genes and were associated with cell motor activity and nucleoside/nucleotide binding. In particular, it was confirmed that the OBSCN gene and the PIK3CA gene constituted a synthetic cancer survival pair of genes in the MSI group. In other words, it was confirmed that, in a pair of OBSCN and PIK3CA, two only-deleteriousness groups in which only OBSCN had a low gene deleteriousness score or only PIK3CA had a low gene deleteriousness score were not significantly different in survival rate of cancer patients compared to none-deleteriousness group both genes did not have low gene deleteriousness scores. But, it was confirmed that both-deleteriousness group in which OBSCN and PIK3CA had low gene deleteriousness scores were statistically significantly higher in the survival rate of cancer patients compared to three other groups (P < 0.05 and HR > 1.0). Therefore, it was confirmed that a pair of OBSCN and PIK3CA genes, which show somatic mutation in colon adenocarcinoma, satisfied the criterion of synthetic cancer survival pair of genes of colon adenocarcinoma as defined above.

Next, as in Example 3, the effect of the number of synthetic cancer survival pairs of genes on the prognosis and survival rate of cancer patients was analyzed. The results are shown in Table 4.

[Table 4]

|  | Alive | Death | Total |
|---|---|---|---|
| SCS pair = 0 | 288 | 57 | 345 |
| SCS pair > 0 | 82 | 0 | 82 |
| Total | 370 | 57 | 427 |

As shown in Table 4, 427 colon adenocarcinoma patients were divided into two groups: 345 persons who did not have any of the synthetic cancer survival pairs of genes and 82 persons who had more than one, and then, the survival analysis was conducted by applying the Cox proportional hazards model. As a result, it was confirmed that the survival rate of 82 persons with the synthetic cancer survival pair of genes was statistically significantly higher (p < 0.0005 and HR > 1.0). These results indicate that survival prognosis of the relevant cancer patients can be predicted by confirming the synthetic cancer survival burden expressed by the number of synthetic cancer survival pair of genes of the cancer patient.

As described above, the above results have a very important medical significance compared to one having no synthetic cancer survival pair of genes found in the analysis of whole colon adenocarcinoma patients without distinguishing MSI status from the same data. It is generally known that when statistical analysis of a larger number of patients, such as using whole colon adenocarcinoma patients was conducted, it is likely to detect significant results. However, this example illustrates that conducting a synthetic cancer survival analysis in a more homogeneous group based on biological markers can provide more accurate results. For example, diagnosis, treatment, and prognosis thereof are significantly affected depending on whether hormone receptors such as an estrogen receptor (ER) and a progesterone receptor (PR) are expressed in breast cancer, and thus these are determined by dividing into subgroups thereof. Therefore, this Example indicates that it is useful and effective to conduct the synthetic cancer survival analysis by dividing the same cancer type into various subgroups according to the latest biological markers, and this method falls within the scope of the present invention.

**Claims**

1.    A method of providing information for customized anticancer therapeutic drug selection using a genomic nucleotide sequence variation of a cancer patient, the method comprising:

   determining gene nucleotide sequence variant information of at least one gene belonging to a synthetic cancer survival pair of genes from the genomic nucleotide sequence information of the cancer patient; and
   selecting at least one candidate drug which inhibits at least one corresponding gene pairing with the at least one variant gene belonging to the synthetic cancer survival pair of genes from the gene nucleotide sequence variant information.

2.    The method of claim 1, wherein the gene nucleotide sequence variant information is for substitution, addition, or deletion of a base constituting an exon of the gene.

3.    The method of claim 2, wherein the substitution, addition, or deletion of the base is by structural abnormality including chromosomal cleavage, deletion, duplication, inversion, or translocation.

4.    The method of claim 1, wherein the gene nucleotide sequence variant information is obtained by a comparative analysis with a genomic nucleotide sequence of a reference group.

5.    The method of claim 1, wherein the variant gene and the corresponding gene are calculated based on the presence or absence of a loss of function (LoF) variant.

6. The method of claim 1, wherein the variant gene and the corresponding gene are determined by a gene nucleotide sequence variant score included in each relevant gene.

7. The method of claim 1, wherein the variant gene and the corresponding gene are calculated from at least one gene nucleotide sequence variant score calculated by applying at least one algorithm selected from the group consisting of SIFT (Sorting Intolerant From Tolerant), PolyPhen, PolyPhen-2 (Polymorphism Phenotyping), MAPP (Multivariate Analysis of Protein Polymorphism), Logre (Log R Pfam E-value), Mutation Assessor, Condel, GERP (Genomic Evolutionary Rate Profiling), CADD (Combined Annotation-Dependent Depletion), MutationTaster, MutationTaster2, PROVEAN, PMuit, CEO (Combinatorial Entropy Optimization), SNPeffect, fathmm, MSRV (Multiple Selection Rule Voting), Align-GVGD, DANN, Eigen, KGGSeq, LRT (Likelihood Ratio Test), MetaLR, MetaSVM, MutPred, PANTHER, Parepro, phastCons, PhD-SNP, phyloP, PON-P, PON-P2, SiPhy, SNAP, SNPs&GO, VEP (Variant Effect Predictor), VEST (Variant Effect Scoring Tool), SNAP2, CAROL, PaPI, Grantham, SInBaD, VAAST, REVEL, CHASM (Cancer-specific High-throughput Annotation of Somatic Mutations), mCluster, nsSNPAnayzer, SAAPpred, HanSa, CanPredict, FIS, and BONGO (Bonds ON Graphs) to the gene nucleotide sequence variant included in each relevant gene.

8. The method of claim 1, wherein the variant gene and the corresponding gene are determined by a gene deleteriousness score calculated from at least one gene nucleotide sequence variant score calculated by applying at least one algorithm selected from the group consisting of SIFT (Sorting Intolerant From Tolerant), PolyPhen, PolyPhen-2 (Polymorphism Phenotyping), MAPP (Multivariate Analysis of Protein Polymorphism), Logre (Log R Pfam E-value), Mutation Assessor, Condel, GERP (Genomic Evolutionary Rate Profiling), CADD (Combined Annotation-Dependent Depletion), MutationTaster, MutationTaster2, PROVEAN, PMuit, CEO (Combinatorial Entropy Optimization), SNPeffect, fathmm, MSRV (Multiple Selection Rule Voting), Align-GVGD, DANN, Eigen, KGGSeq, LRT (Likelihood Ratio Test), MetaLR, MetaSVM, MutPred, PANTHER, Parepro, phastCons, PhD-SNP, phyloP, PON-P, PON-P2, SiPhy, SNAP, SNPs&GO, VEP (Variant Effect Predictor), VEST (Variant Effect Scoring Tool), SNAP2, CAROL, PaPI, Grantham, SInBaD, VAAST, REVEL, CHASM (Cancer-specific High-throughput Annotation of Somatic Mutations), mCluster, nsSNPAnayzer, SAAPpred, HanSa, CanPredict, FIS, and BONGO (Bonds ON Graphs) to the gene nucleotide sequence variant included in each relevant gene.

9. The method of claim 1, wherein the variant gene and the corresponding gene are, when the gene nucleotide sequence variants included in each relevant gene are at least two, determined by a gene deleteriousness score calculated as a mean value of respective gene nucleotide sequence variant scores.

10. The method of claim 9, wherein the mean value is calculated by at least one selected from the group consisting of a geometric mean, an arithmetic mean, a harmonic mean, an arithmetic-geometric mean, an arithmetic-harmonic mean, a geometric-harmonic mean, a Pythagorean mean, a heron mean, an inverse harmonic mean, a root mean square deviation, a centroid mean, a quartile mean, a quadratic mean, a truncated mean, a winsorized mean, a weighted mean, a weighted geometric mean, a weighted arithmetic mean, a weighted harmonic mean, a function mean, a power mean, a generalized f-mean, percentile, maximum, minimum, mode, median, central range, measures of central tendency, simple product, and weighted product.

11. The method of claim 8, wherein the gene deleteriousness score is calculated by the following Equation 1:

[Equation 1]

$$S_g(v_{1,...,}v_n) = \left(\frac{1}{n}\sum_{i=1}^{n} v_i^p\right)^{\frac{1}{p}}$$

wherein $S_g$ is a gene deleteriousness score of the protein encoded by gene $g$, $n$ is the number of nucleotide sequence variants to be analyzed among the nucleotide sequence variants of the gene $g$, $v_i$ is a nucleotide sequence variant score of $i$-th nucleotide sequence variant to be analyzed, and $p$ is a non-zero real number.

12. The method of claim 8, wherein the gene deleteriousness score is calculated by the following Equation 2:

[Equation 2]

$$S_g(v_{1,...,}v_n) = \left(\prod_{i=1}^{n} v_i^{w_i}\right)^{1/\sum_{i=1}^{n} w_i}$$

wherein $S_g$ is a gene deleteriousness score of the protein encoded by gene $g$, $n$ is the number of nucleotide sequence variants to be analyzed among the nucleotide sequence variants of the gene $g$, $v_i$ is a nucleotide sequence variant score of $i$-th nucleotide sequence variant to be analyzed, and $wi$ is a weight given to the gene nucleotide sequence variant score $vi$ of the $i$-th nucleotide sequence variant.

13. The method of claim 1, wherein the synthetic cancer survival pair of genes refers to a gene pair in which a combination of at least two variant genes included in a cancer cell line or cancer tissue induces an improvement in the survival rate of an relevant cancer patient, and
wherein each individual variant gene among the at least two variant genes does not induce an improvement in the survival rate of the relevant cancer patient, but the combination of the at least two variant genes induces an improvement in the survival rate of the relevant cancer patient.

14. The method of claim 1, the method further comprising: determining a priority of drugs to be applied to the cancer patient using the synthetic cancer survival pair of genes information; or
determining whether to use the drugs to be applied to the cancer patient using the synthetic cancer survival pair of genes information.

15. The method of claim 1, wherein the selection of the synthetic cancer survival pair of genes including at least one gene for determining the nucleotide sequence variant information is carried out by conducting a cancer patient survival analysis using genetic mutant and survival information of cancer patients; or
conducting a genomic mutant analysis of a cancer cell line or cancer tissue and an identification of invasive or metastatic ability of the cancer cell line or cancer tissue.

16. The method of claim 15, wherein the cancer patient survival analysis is conducted in which the cancer patients are divided into at least two subgroups based on a biological marker, and then the genomic mutant information and patient survival information in each subgroup are used.

17. The method of claim 1, wherein the selection of the candidate drug is performed by calculating the number of at least one variant gene pairing with at least one corresponding gene belonging to the synthetic cancer survival pair of genes selected from the genomic nucleotide sequence information of the cancer patient to determine the priority or combination of candidate drugs based on the calculated number.

18. A system for selecting a customized anticancer therapeutic drug using genomic nucleotide sequence variant information of a cancer patient, the system comprising:

a database in which information related to an anticancer therapeutic drug to be applied to cancer patients and a gene inhibited by the drug is searched or extracted;
a communication unit accessible to the database;
a cancer genomic nucleotide sequence analyzer;
a drug selection information provider; and
a display,
wherein the cancer genomic nucleotide sequence analyzer includes: a variant gene selector selecting at least one variant gene belonging to a synthetic cancer survival pair of genes; and a corresponding gene selector selecting at least one corresponding gene pairing with the relevant at least one variant gene constituting the synthetic cancer survival pair of genes, and
wherein the drug selection information provider provides anticancer therapeutic drug selection information for inhibiting the relevant at least one corresponding gene.

19. A computer-readable medium comprising an executable module for executing the following processor:

the processor executing an operation comprising:

selecting a synthetic cancer survival pair of genes from genomic nucleotide sequence information of a cancer patient; and
selecting at least one candidate drug that inhibits at least one corresponding gene pairing with at least one variant gene belonging to the synthetic cancer survival pair of genes.

20. A method of providing information for predicting prognosis of a cancer patient, the method comprising calculating the number of at least one gene belonging to the synthetic cancer survival pair of genes from nucleotide sequence information of a cancer patient genome.

21. The method of claim 20, comprising calculating the number of the at least one gene belonging to the synthetic cancer survival pair of genes and the number of somatic mutant gene from the nucleotide sequence information of the cancer patient genome.

22. A system for selecting a customized anticancer therapeutic drug using genomic nucleotide sequence variant information of a cancer patient, the system comprising:

a database in which information related to an anticancer therapeutic drug to be applied to cancer patients and a gene inhibited by the drug is searched or extracted;
a communication unit accessible to the database;
a cancer genomic nucleotide sequence analyzer;
a drug selection information provider; and
a display,
wherein the cancer genomic nucleotide sequence analyzer includes: a variant gene pair selector selecting at least one variant gene belonging to a synthetic cancer survival pair of genes; and a corresponding gene selector selecting at least one corresponding gene pairing with the relevant at least one variant gene constituting the synthetic cancer survival pair of genes, and
wherein the drug selection information provider provides drug selection information for increasing the number of synthetic cancer survival pairs of genes of the cancer patient.

23. A computer-readable medium comprising an executable module for executing the following processor:

the processor executing an operation comprising:

selecting a synthetic cancer survival pair of genes from genomic nucleotide sequence information of a cancer patient; and
selecting a candidate drug that increases the number of synthetic cancer survival pairs of genes among at least one candidate drug that inhibits at least one corresponding gene pairing with at least one variant gene belonging to the synthetic cancer survival pair of genes.

Fig. 1

DNAH2_XIRP2 (0.03634,0.01910, 0.02141)

Year
SKCM

Both <= 0.3:1/12 death (8.3%)
DNAH2 only <= 0.3:7/15 death (46.7%)
XIRP2 only <= 0.3:11/28 death (39.3)
None <= 0.3:55/118 death (46.6%)

Fig. 2

LUAD
SKCM
LUSC
HNSC
KIRP

Fig. 3

EP 3 396 573 A2

Fig. 4

Fig. 5

EP 3 396 573 A2

Fig. 6

Fig. 7

# Fig. 8

LUAD (n = 341) r = 0.889

SKCM (n = 181) r = 0.705

# Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020140107916
- KR 2014007685 W

### Non-patent literature cited in the description

- **WANG et al.** *Nucleic Acids Research,* 2010, vol. 38 (16), e164
- **GE et al.** *Bioinformatics,* 2011, vol. 27 (14), 1998-2000
- **CHEN et al.** *Nat Methods,* September 2009, vol. 6 (9), 677-81
- **PAULINE C et al.** Sorting Intolerant From Tolerant. *Genome Res.,* May 2001, vol. 11 (5), 863-874
- **PAULINE C et al.** *Genome Res.,* March 2002, vol. 12 (3), 436-446
- **JING HUL et al.** *Genome Biol.,* 2012, vol. 13 (2), R9
- **RAMENSKY V et al.** *Nucleic Acids Res.,* 01 September 2002, vol. 30 (17), 3894-3900
- **ADZHUBEI IA et al.** *Nat Methods,* 2010, vol. 7 (4), 248-249
- **ERIC A. et al.** Multivariate Analysis of Protein Polymorphism. *Genome Res.,* 2005, vol. 15, 978-986
- **CLIFFORD R.J et al.** Log R Pfam E-value. *Bioinformatics,* 2004, vol. 20, 1006-1014
- **REVA B et al.** *Genome Biol.,* 2007, vol. 8, R232, http://mutationassessor.org
- **GONZALEZ-PEREZ A et al.** *The American Journal of Human Genetics,* 2011, vol. 88, 440-449, http://bg.upf.edu/fannsdb
- **COOPER et al.** Genomic Evolutionary Rate Profiling. *Genome Res.,* 2005, vol. 15, 901-913, http://mendel.stanford.edu/SidowLab/downloads/gerp
- **SCHWARZ et al.** MutationTaster2: mutation prediction for the deep-sequencing age. *Nature Methods,* 2014, vol. 11, 361-362, http://www.mutationtaster.org
- **CHOI et al.** *PLoS One,* 2012, vol. 7 (10), e46688
- **FERRER-COSTA et al.** *Proteins,* 2004, vol. 57 (4), 811-819, http://mmb.pcb.ub.es/PMut
- **REVA et al.** Combinatorial Entropy Optimization. *Genome Biol,* 2007, vol. 8 (11), R232
- **REUMERS et al.** *Bioinformatics,* 2006, vol. 22 (17), 2183-2185, http://snpeffect.vib.be
- **SHIHAB et al.** Functional Analysis through Hidden Markov Models. *Hum Mutat,* 2013, vol. 34, 57-65, http://fathmm.biocompute.org.uk
- **JIANG, R. et al.** Sequence-based prioritization of nonsynonymous single-nucleotide polymorphisms for the study of disease mutations. *Am J Hum Genet,* 2007, vol. 81, 346-360, http://msms.usc.edu/msrv
- **TAVTIGIAN, SEAN V. et al.** Comprehensive statistical study of 452 BRCA1 missense substitutions with classification of eight recurrent substitutions as neutral. *Journal of medical genetics,* 2006, 295-305, http://agvgd.hci.utah.edu
- **QUANG, DANIEL ; YIFEI CHEN ; XIAOHUI XIE.** DANN: a deep learning approach for annotating the pathogenicity of genetic variants. *Bioinformatics,* 2014, btu703, https://cbcl.ics.uci.edu/public_data/DANN
- **IONITA-LAZA, IULIANA et al.** A spectral approach integrating functional genomic annotations for coding and noncoding variants. *Nature genetics,* 2016, 214-220, http://www.columbia.edu/~ii2135/eigen.html
- **LI MX ; GUI HS ; KWAN JS ; BAO SY ; SHAM PC.** A comprehensive framework for prioritizing variants in exome sequencing studies of Mendelian diseases. *Nucleic Acids Res.,* April 2012, vol. 40 (7), e53, http://grass.cgs.hku.hk/limx/kggseq
- **CHUN, SUNG ; JUSTIN C. FAY.** Identification of deleterious mutations within three human genomes. *Genome Res.,* 2009, 1553-1561, http://www.genetics.wustl.edu/jflab/lrt_query.html
- **DONG, CHENGLIANG et al.** Comparison and integration of deleteriousness prediction methods for nonsynonymous SNVs in whole exome sequencing studies. *Human molecular genetics,* 2015, vol. 24 (8), 2125-2137
- **MORT, MATTHEW et al.** MutPred Splice: machine learning-based prediction of exonic variants that disrupt splicing. *Genome Biology,* 2014, vol. 15 (1), 1, http://www.mutdb.org/mutpredsplice/about.htm
- **MI, HUAIYU et al.** The PANTHER database of protein families, subfamilies, functions and pathways. *Nucleic Acids Research,* 2005, vol. 33 (1), D284-D288, http://www.pantherdb.org/tools/csnpScoreForm.jsp

- **TIAN, JIAN et al.** Predicting the phenotypic effects of non-synonymous single nucleotide polymorphisms based on support vector machines. *BMC bioinformatics,* 2007, vol. 8.1, http://www.mobioinfor.cn/parepro/contact.htm
- **SIEPEL, ADAM et al.** Evolutionarily conserved elements in vertebrate, insect, worm, and yeast genomes. *Genome Res.,* 2005, vol. 915 (8), 1034-1050, http://compgen.cshl.edu/phast
- **CAPRIOTTI, E. ; CALABRESE, R. ; CASADIO, R.** Predicting the insurgence of human genetic diseases associated to single point protein mutations with support vector machines and evolutionary information. *Bioinformatics,* 2006, vol. 22, 2729-2734, http://snps.biofold.org/phd-snp
- **POLLARD, KATHERINE S. et al.** Detection of nonneutral substitution rates on mammalian phylogenies. *Genome Res.,* 2010, vol. 20 (1), 110-121, http://compgen.cshl.edu/phast/background.php
- **NIROULA, ABHISHEK ; SIDDHALING UROLAGIN ; MAUNO VIHINEN.** PON-P2: prediction method for fast and reliable identification of harmful variants. *PLoS One,* 2015, vol. 10 (2), e0117380, http://structure.bmc.lu.se/PON-P2
- **GARBER, MANUEL et al.** Identifying novel constrained elements by exploiting biased substitution patterns. *Bioinformatics,* 2009, vol. 25 (12), i54-i62, http://portals.broadinstitute.org/genome_bio/siphy/documentation.html
- **BROMBERG,Y. ; ROST,B.** SNAP: predict effect of non-synonymous polymorphisms on function. *Nucleic Acids Res.,* 2007, vol. 35, 3823-3835, http://www.rostlab.org/services/SNAP
- **REMO CALABRESE ; EMIDIO CAPRIOTTI ; PIERO FARISELLI ; PIER LUIGI MARTELLI ; RITA CASADIO.** Functional annotations improve the predictive score of human disease-related mutations in proteins. *Human Mutation,* 2009, vol. 30, 1237-1244, http://snps.biofold.org/snps-and-go
- **MCLAREN W ; PRITCHARD B ; RIOS D ; CHEN Y ; FLICEK P ; CUNNINGHAM F.** Deriving the consequences of genomic variants with the Ensembl API and SNP Effect Predictor. *Bioinformatics,* 2010, vol. 26, 2069-70, http://www.ensembl.org/info/docs/tools/vep
- **CARTER H ; DOUVILLE C ; STENSON P ; COOPER D ; KARCHIN R.** Identifying Mendelian disease genes with the Variant Effect Scoring Tool. *BMC Genomics,* 2013, vol. 14 (3), S3
- **YANA BROMBERG ; GUY YACHDAV ; BURKHARD ROST.** SNAP predicts effect of mutations on protein function. *Bioinformatics,* 2008, vol. 24, 2397-2398, http://www.rostlab.org/services/SNAP
- **LOPES MC ; JOYCE C ; RITCHIE GR ; JOHN SL ; CUNNINGHAM F et al.** *A combined functional annotation score for non-synonymous variants, http://www.sanger.ac.uk/science/tools/carol*
- **LIMONGELLI, IVAN ; SIMONE MARINI ; RICCARDO BELLAZZI.** PaPI: pseudo amino acid composition to score human protein-coding variants. *BMC bioinformatics,* 2015, vol. 16 (1), 1, http://papi.unipv.it
- **GRANTHAM, R.** Amino acid difference formula to help explain protein evolution. *Science,* 1974, vol. 185 (4154), 862-864, https://ionreporter.thermofisher.com/ionreporter/help/GUID-D9DFB21C-652D-4F95-8132-A0C442F65399.html
- **LEHMANN ; KJONG-VAN ; TING CHEN.** Exploring functional variant discovery in non-coding regions with SInBaD. *Nucleic Acids Research,* 2013, vol. 41 (1), e7-e7, http://tingchenlab.cmb.usc.edu/sinbad
- **HU, HAO et al.** VAAST 2.0: Improved variant classification and disease_]gene identification using a conservation_]controlled amino acid substitution matrix. *Genetic epidemiology,* 2013, vol. 37 (6), 622-634, http://www.yandelllab.org/software/vaast.html
- **NILAH M. et al.** REVEL: an Ensemble Method for Predicting the Pathogenicity of Rare Missense Variants. *AGHG,* 2016, https://sites.google.com/site/revelgenomics
- **CARTER H ; CHEN S ; ISIK L ; TYEKUCHEVA S ; VELCULESCU VE ; KINZLER KW ; VOGELSTEIN B.** Karchin R Cancer-specific high-throughput annotation of somatic mutations: computational prediction of driver missense mutations. *Cancer Res,* 2009, vol. 69 (16), 6660-7, http://www.cravat.us
- **YUE P ; FORREST WF ; KAMINKER JS ; LOHR S ; ZHANG Z ; CAVET G.** Inferring the functional effects of mutation through clusters of mutations in homologous proteins. *Human mutation.,* 2010, vol. 31 (3), 264-271, 10.1002/humu.21194
- **LEI BAO ; MI ZHOU ; YAN CUI.** nsSNPAnalyzer: identifying disease-associated nonsynonymous single nucleotide polymorphisms. *Nucleic Acids Res,* 2005, vol. 33, 480-482, http://snpanalyzer.uthsc.edu
- **NOUF S AL-NUMAIR ; ANDREW C R MARTIN.** The SAAP pipeline and database: tools to analyze the impact and predict the pathogenicity of mutations. *BMC Genomics,* 2013, vol. 14 (3), 1-11, www.bioinf.org.uk/saap/dap
- **ACHARYA V. ; NAGARAJARAM H.A.** Hansa An automated method for discriminating disease and neutral human nsSNPs. *Human Mutation,* 2012, vol. 2, 332-337, hansa.cdfd.org.in:8080

- **KAMINKER,J.S. et al.** CanPredict: a computational tool for predicting cancer-associated missense mutations. *Nucleic Acids Res.,* 2007, vol. 35 (595), 598, http://pgws.nci.nih.gov/cgi-bin/GeneViewer.cgi
- **BORIS REVA ; YEVGENIY ANTIPIN ; CHRIS SANDER.** Predicting the functional impact of protein mutations: Application to cancer genomics. *Nucleic Acids Res,* 2011, vol. 39, e118-e118
- **CHENG T.M.K. ; LU Y-E ; VENDRUSCOLO M. ; LIO P. ; BLUNDELL T.L.** Prediction by graph theoretic measures of structural effects in proteins arising from non-synonymous single nucleotide polymorphisms. *PLoS Comp Biology,* 2008, vol. 4 (7), e1000135, http:// www.bongo.cl.cam.ac.uk/Bongo2/Bongo.htm
- **GONZALEZ-PEEREZ, A. ; LOPEZ-BIGAS, N.** Improving the assessment of the outcome of nonsynonymous SNVs with a consensus deleteriousness score, Condel. *The American Journal of Human Genetics,* 2011, vol. 88 (4), 440-449
- **ADZHUBEI, IA et al.** A method and server for predicting damaging missense mutations. *Nature Methods,* 2010, vol. 7 (4), 248-249
- **ZHANG et al.** *Proc Natl Acad Sci U S A.,* 16 August 2011, vol. 108 (33), 13653-13658